# EUROPEAN PATENT SPECIFICATION

(11) **EP 4 241 275 B1**
(45) Date of publication and mention of the grant of the patent: **09.10.2024**
(21) Application number: 21830820.3
(22) Date of filing: 05.11.2021
(51) Int. Cl.: G16B 25/10, G16B 40/30

(54) **SYSTEMS AND METHODS FOR PRE-HARVEST DETECTION OF LATENT INFECTION IN PLANTS**
SYSTEME UND VERFAHREN ZUR VOR DER ERNTE ERFOLGENDEN ERKENNUNG LATENTER INFEKTIONEN IN PFLANZEN
SYSTÈMES ET PROCÉDÉS DE DÉTECTION AVANT RÉCOLTE D'UNE INFECTION LATENTE CHEZ DES PLANTES

(30) Priority: 05.11.2020 US 202063110343 P
(43) Date of publication of application: 13.09.2023
(73) Proprietor: Apeel Technology, Inc., Goleta, California 93117 (US)
(72) Inventor: VILD, Cody, Santa Barbara, California 93108 (US); BRADEN, Savannah, Goleta, California 93117 (US); KAHLSCHEUER, Matthew, Goleta, California 93117 (US); PEREZ, Louis, Santa Barbara, California 93105 (US)
(74) Representative: Elkington and Fife LLP
(86) International application number: PCT/US2021/058212
(87) International publication number: WO 2022/098976

(56) References cited:
- WO-A1-2021/092251
- WENNEKER MARCEL ET AL: "Latent postharvest pathogens of pome fruit and their management: from single measures to a systems intervention approach", EUROPEAN JOURNAL OF PLANT PATHOLOGY, SPRINGER NETHERLANDS, NL, vol. 156, no. 3, 21 January 2020 (2020-01-21), pages 663 - 681, XP037074451, ISSN: 0929-1873, [retrieved on 20200121], DOI: 10.1007/S10658-020-01935-9
- TIWARI SHIKHA ET AL: "Volatile organic compounds (VOCs): Biomarkers for quality management of horticultural commodities during storage through e-sensing", TRENDS IN FOOD SCIENCE AND TECHNOLOGY, ELSEVIER SCIENCE PUBLISHERS, GB, vol. 106, 29 October 2020 (2020-10-29), pages 417 - 433, XP086399114, ISSN: 0924-2244, [retrieved on 20201029], DOI: 10.1016/J.TIFS.2020.10.039
- ASCENCIO-IBáñEZ JOSé TRINIDAD ET AL: "Global Analysis of Arabidopsis Gene Expression Uncovers a Complex Array of Changes Impacting Pathogen Response and Cell Cycle during Geminivirus Infection", PLANT PHYSIOLOGY, vol. 148, no. 1, 23 July 2008 (2008-07-23), Rockville, Md, USA, pages 436 - 454, XP055772257, ISSN: 0032-0889, DOI: 10.1104/pp.108.121038
- USHA DEVI GANDHI ET AL: "A Survey on Plant Disease Prediction using Machine Learning and Deep Learning Techniques A Survey on Plant Disease Prediction using Machine Learning and Deep Learning Techniques", INTELIGENCIA ARTIFICIAL, 1 January 2017 (2017-01-01), pages 0 - 19, XP055771922, Retrieved from the Internet <URL:https://www.researchgate.net/publication/343501078_A_Survey_on_Plant_Disease_Prediction_using_Machine_Learning_and_Deep_Learning_Techniques> [retrieved on 20220201], DOI: 10.4114/intartif.vol22iss63pp0-19

## Description

### TECHNICAL FIELD

This document describes devices, systems, and methods related to pre-harvest prediction of latent infection in different types of plants (e.g., produce).

### BACKGROUND

Many plant products, for example, fruits, can sustain high rates of infection. Pre-harvest infection of plant products can occur when a plant product becomes infected by a pathogen during growth. For instance, approximately 20-40% of avocado trees in avocado orchards, and thus the avocados that they produce, can become infected with anthracnose pre-harvest. Unripe, pre-harvest plant products can appear asymptomatic of such infection. However, after a plant product has been harvested, ripens, and undergoes senescence, the plant product can become symptomatic of the infection. Symptoms of infection can include stem-end rot, mold, vascular/internal browning, and other features that may result in a detrimental loss in quality of the plant product.

Harvest, for example, can involve excision of fruit from a parent tree through plucking or clipping, both of which may expose xylem elements of a pedicel of the fruit to infection, which can facilitate transition of fungal inoculum into the xylem tissue of the fruit. While the fruit remains unripe, the fungi may colonize the xylem of the fruit but remain latent, and may aggressively invade surrounding tissue, causing cell damage and disease symptoms, as the fruit ripens. This makes infections such as stem end rot (SER) a challenging post-harvest disorder: it originates on the farm yet may not be detectable until the plant product (e.g., produce) is in the hands of a consumer. Moreover, infections such as SER may have severity that is temporally and spatially dependent. This means that an early season harvest can have a different incidence of SER than a late season harvest from the same tree and this incidence may not necessarily be comparable across neighboring trees.

Some pre- and post-harvest mitigation methods can include applying fungicide chemicals pre- and/or post-harvest. Repeated fungicide application can have negative consequences for the environment, including deterioration of beneficial microbiota of parent trees. The repeated fungicide application can also lead to development of resistant strains of pathogens over time.

Wenneker and Thomma, Eur J Plant Pathol (2020) discusses postharvest diseases of pome fruit and considers systems intervention approaches for their control. Tiwari et al., Trends in Food Science and Technology (2020) describes volatile organic compounds emitted by plants, and considers techniques involving these compounds for the detection of the quality of horticulture crops in commercial storage. Ascencio-Ibáñez et al., Plant Physiology (2008) provides a transcriptome analysis of the Arabidopsis plant in response to the cabbage leaf curl virus, and demonstrates altered expression in various cellular mechanisms. U Gandhi and BV Gokulnath, Inteligencia Artificial (2020) describes machine learning techniques used in the automatic detection of plant disease from visual symptoms in the plant, for early diagnosis and prevention of disease.

### SUMMARY

This disclosure generally relates to pre-harvest evaluation of plants (e.g., produce) to predict occurrence of latent infections in fruits, vegetables, seeds, and/or other plant products (e.g., leaves, stems, roots, etc.) that grow or otherwise are derived from the evaluated plants. More specifically, machine learning models can be used to predict likelihood of latent infection in plants using pre-harvest data about the plants and prediction features. The prediction features can include but are not limited to geographic region of the plants, stage of growth, age, hormones, dry matter content, environmental conditions, etc.

In an exemplary implementation of the disclosed techniques, by surveying fruit from the same trees throughout a season, a dataset of physiological and transcriptomic information can be built profiling trees that produce fruit with high or low incidence of one or more latent infections, such as stem end rot (SER). Although such latent infections of fruit, including but not limited to avocados, may not manifest until the fruit reaches maturity on the tree and ripened post-harvest, the fruit can harbor markers of infection as early as 6 months prior to peak harvest season. Thus, the disclosed techniques can provide a platform for using predictive assays in pre-harvest detection of latent infections in plants with ample time to make amendments (e.g., mitigation operations) to fungicide applications, plant harvest schedule, and/or plant market destination efforts to mitigate manifestation of such latent infections in the plants.

The invention is set out in the appended claims and, in one aspect, relates to a method for identifying pre-harvest latent infection in a plant, the method comprising: obtaining, by a processor, data describing a level of expression of one or more infection biomarkers present in the plant, the data including a list of one or more variants, the infection biomarkers indicating a likelihood of infection in the plant, wherein the one or more variants describe differences between a read sequence of the plant and a reference genome of a healthy plant of a same type as the plant, wherein the read sequence of the plant represents an order of nucleotides in a nucleic acid of the plant; selecting, by the processor, one or more machine learning models based on the obtained data, wherein the one or more machine learning models were previously trained, using data that correlates other data and determined one or more infection biomarkers of one or more other plants, to generate output indicating a likelihood that the plant is developing a latent infection pre-harvest, wherein the one or more machine learning models were trained using a process comprising: inputting, from a training data set and into the one or more machine learning models, (i) non-invasive measurements of the one or more other plants, (ii) invasive measurements of the one or more other plants, (iii) known plant information for the one or more other plants, and (iv) affirmative infection identifications for the one or more other plants, and determining predicted likelihoods that the one or more other plants are developing the latent infection pre-harvest based on inputting (i)-(iv) into the one or more machine learning models, and outputting the one or more machine learning models for runtime use; generating, by the processor, output indicating the likelihood that the plant is developing the latent infection pre-harvest based on applying the one or more machine learning models to the data; determining, by the processor, that the plant has a latent infection based on the output exceeding a predetermined threshold range; determining, by the processor, one or more operations to mitigate the latent infection in the plant; and outputting, by the processor, an indication that the plant has the latent infection and the one or more determined operations.

Also provided is a system for predicting a latent infection in a plant, the system comprising one or more processors; and one or more computer-readable storage devices having stored thereon instructions that, when executed by the one or more processors, cause the one or more processors to perform operations according to the method set out above.

Other aspects can include corresponding systems, apparatus, and computer programs to perform the actions of methods as disclosed herein, as defined by instructions encoded on computer readable storage devices. These and other aspects may optionally include one or more of the following features. For example, performing, by the one or more computers, the one or more operations to mitigate the latent infection in the plant can include determining, by the one or more computers, that a harvest schedule for the plant and one or more other similarly sourced plants is to be adjusted, and storing, by the one or more computers, data in a memory device that defines the adjusted harvest schedule.

In some implementations, performing, by the one or more computers, the one or more operations to mitigate the latent infection in the plant can include generating, by the one or more computers, an alert message that, when processed by a user device, can cause the user device to output an alert that notifies a user of the user device that the plant and one or more other similarly sourced plants are to be harvested, and transmitting, by the one or more computers, the generated alert to the user device. Performing, by the one or more computers, the one or more operations to mitigate the latent infection in the plant can also include determining, by the one or more computers and based on the output data, that an anti-microbial treatment can be prescribed for one or more other similarly sourced plants.

In some implementations, the one or more other similarly sourced plants can include plants within the same zone as the plant. Moreover, performing, by the one or more computers, the one or more operations to mitigate the latent infection in the plant can include generating, by the one or more computers, one or more instructions that, when processed by an irrigation controller, can cause the irrigation controller to disperse a liquid including the anti-microbial treatment, and transmitting, by the one or more computers, the one or more instructions to the irrigation controller using one or more networks. As yet another example, performing, by the one or more computers, the one or more operations to mitigate the latent infection in the product can include generating, by the one or more computers, one or more instructions that, when processed by a robotic device, can cause the robotic device to (i) navigate to a location associated with the plant and (ii) initiate antimicrobial operations, by the robotic device, that can cause the robotic device to disperse a liquid include an antimicrobial treatment onto the plant and one or more other locally sourced plants, and transmitting, by the one or more computers, the one or more instructions to the robotic device using one or more networks.

In some implementations, performing, by the one or more computers, the one or more operations to mitigate the latent infection in the product can include generating, by the one or more computers, one or more instructions that, when processed by a robotic device, can cause the robotic device to (i) navigate to a location associated with the plant and (ii) initiate harvesting operations, by the robotic device, that can cause the robotic device to harvest a plant product from the plant and one or more other similarly sourced plants, and transmitting, by the one or more computers, the one or more instructions to the robotic device using one or more networks.

In some implementations, the obtained data can be generated based on output data generated by a nucleic acid sequencer based on the nucleic acid sequencer's sequencing of (i) a sample of bark extracted from the plant or (ii) a sample of a plant product extracted from a plant product of the plant. Sometimes, the obtained data can be generated based on output data generated by a nucleic acid sequencer based on the nucleic acid sequencer's sequencing of (i) a sample of bark extracted from the plant and (ii) a sample of a plant product extracted from a plant product of the plant. In some implementations, the machine learning model can include one or more of a binary logistic regression model, logistic model tree, random forest classifier, L2 regularization, partial least squares, or one or more neural networks. Sometimes, the plant product may not include any visible signs of an infection.

The devices, system, and techniques described herein may provide one or more of the following advantages. For example, early detection of infected plant products can enable treatment and segregation of the infected plant products from uninfected plant products. This can reduce spread of infection, reduce plant product waste, and also improve customer acceptance of the plant products. By reducing plant product waste and improving customer acceptance, providers and other relevant users in the supply chain can provide a higher quality of plant products, thereby enabling the providers to differentiate themselves from other providers in the marketplace. The disclosed techniques can further provide for determining effective mitigation efforts that can be applied early on in plant growth to avoid infection development and plant product waste. Early establishment of latent infection development can provide relevant stakeholders, such as farmers, with amply time to determine and apply mitigation efforts. Furthermore, sampling plants in advance, pre-harvest, may not burden farmers, even if a plant (e.g., a tree) loses some plant products for sampling, since mitigation efforts can be determined and applied to remove or lower possibility of losing plant products during the plants' maturation process.

As another example, the disclosed techniques provide for combining different data and/or features about the plant products, which can be used to make accurate and timely pre-harvest predictions of latent infection. A human may not be as effective nor accurate in collecting and assessing different types of data about plants during their growing season, such as invasively collected measurements and non-invasively collected measurements, to predict development of latent infection. Thus, the disclosed techniques provide for collection, synthesis, and analysis of different types of data, which otherwise may be challenging, erroneous, and/or impossible to do by humans to accurately predict latent infection in plants pre-harvest.

Moreover, the present disclosure can reduce an amount of plant product that needs to be destroyed to detect latent infection in a zone of similarly sourced plants. In some implementations, the present disclosure can reduce destruction of plant products by only sampling an aerial portion of a plant rather than sampling the plant product itself. Then, operations of the present disclosure can be performed using the sample of the aerial portion of the plant. In these, or other implementations, the present disclosure can also provide the benefit of saving plant products by detecting the latent infection in the plant and initiating one or more remedial measures to mitigate, and potentially eliminate, the latent infection before it manifests, thus allowing for the plant product to be sold and improving customer satisfaction. Such remedial measures may include, for example, instructing a user or robotic device to harvest plant product(s) from a plant and plant product(s) from one or more other plants within a zone of similarly sourced plants. In other implementations, the harvesting schedule can also be updated to achieve the same or similar benefits. This can provide one or more economic benefits to users of the present disclosure.

The details of one or more implementations are set forth in the accompanying drawings and the description below.

### BRIEF DESCRIPTION OF THE DRAWINGS

FIG. 1A is a contextual diagram of an example system for detecting latent infection in plants.
FIG. 1B is a flowchart of a process for detecting latent infection in plants.
FIG. 2 is a conceptual diagram for predicting likelihood of infection in plants.
FIG. 3 is a conceptual diagram for training one or more models for predicting the likelihood of infection in plants.
FIG. 4 is a flowchart of a process for predicting likelihood of infection in plants.
FIG. 5 is a system diagram of one or more components that can be used to perform the techniques described herein.
FIGs. 6A-C depict an example process for collecting data of plants to use with the disclosed techniques.
FIGs. 7A-B depict graphical depictions of example physical attributes of the plants of FIG. 6 that can be collected at different times.
FIGs. 8A-C depict biplots of principal component analysis of normalized gene expressions across all times of data collection for the plants of FIGs. 6A-C.
FIGs. 9A-C depict volcano plots of normalized gene expressions for the plants of FIGs. 6A-C.
FIGs. 10A-D depict clustered heatmaps of normalized gene expressions for genes that were analyzed at each collection time for the plants of FIGs. 6A-C.
FIG. 11 is a block diagram of system components that can be used to implement a system for detecting latent infection.

Like reference symbols in the various drawings indicate like elements.

### DETAILED DESCRIPTION OF ILLUSTRATIVE EMBODIMENTS

The present disclosure is generally directed to systems, methods, and computer programs for detecting latent infections in plants. Many plants can regularly produce fruit and live productively for decades to centuries. Throughout this long lifetime, the plants can interact with and respond to their surrounding ecosystem, which can include a population of beneficial and pathogenic microbes. A plant's detection and response to a pathogenic microbe can be observed through changes in the expression of genes involved in the innate immune system of the plant. The disclosed techniques therefore can provide for investigation of pre-harvest transcriptomic markers of manifestation of post-harvest latent infection, such as stem end rot (SER), by profiling the transcriptome of various plant products from different types of plants, such as fruit from trees. Although such latent infections may not manifest until the fruit reaches maturity on the tree, were harvested, and/or ripened, the fruit and other plant products can harbor markers of infection as early as 6 months prior to peak harvest season. Leveraging this information towards production of detection assays can enable prediction of high and low incidence trees with ample time to make amendments to fungicide applications, plant product harvest schedule(s), and/or plant product market destination information to mitigate manifestation of latent infection in different types of plants.

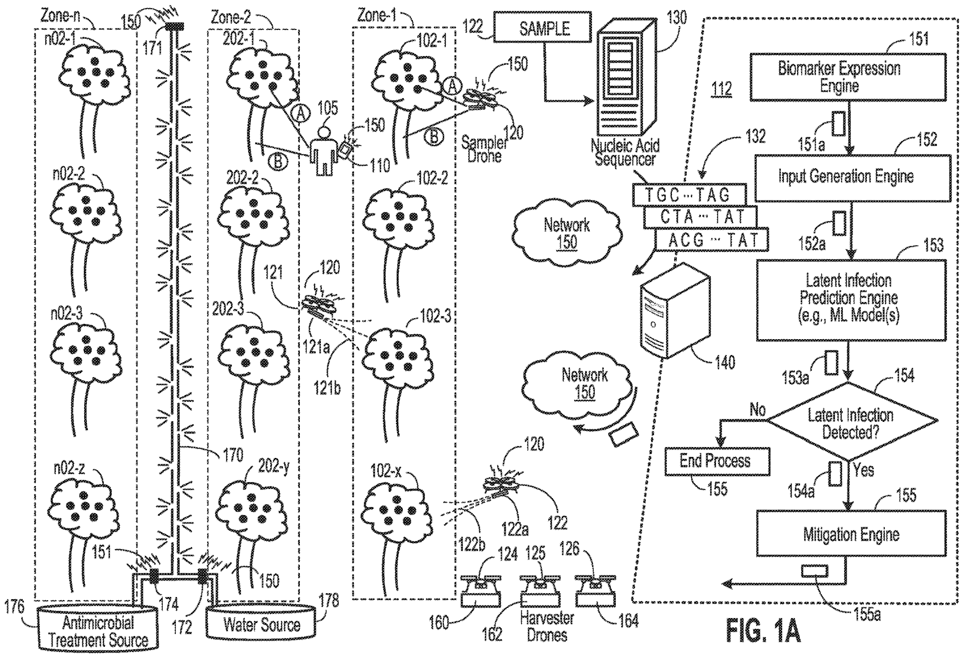

Referring to the figures, FIG. 1A is a contextual diagram of an example system 100 for detecting latent infection in plants. The system 100 can include a user device 110, one or more robotic devices 120, 121, 122, 124, 125, 126, a nucleic acid sequencer 130, a computer 140 (e.g., computer system), a network 150, one or more robotic device charging stations 160, 162, 164, an irrigation system 170, and/or any combination thereof. The system 100 can be implemented with any subset, combination, or arrangement of these components to realize the functionality described herein to detect latent infections in plants, plant products, or both. In some implementations, a nucleic acid sequencer may not be required. Instead, a qPCR machine may be used in place of the sequencer 130. In yet some implementations, the sequencer 130 and the computer 140 can be part of a same system.

With reference to FIG. 1A, execution of the system 100 can be, in some implementations, with user 105 extracting a sample 122 of plant product of plant 202-1 of zone-2, a sample of any aerial portion of the of the plant 202-1, or both. Sampling can occur pre-harvest, which can sometimes be 6 or more months before plant product of the sampled plants is collected and harvested. Thus, the disclosed techniques can be performed while the sampled plants are undergoing their respective maturation processes and before they are harvested (e.g., reach full maturation). As a result, the disclosed techniques can be performed to predict likelihood of infection in the sampled plants and other similarly sourced plants pre-harvest, early enough such that efforts to mitigate potential development of latent infection can be executed.

The sample 122 can include genetic data of the plant product of the sampled plants. In some implementations, the sample 122 can be derived from fruit of the plant 202-1, leaves, bark, plant tissue(s), or other plant products of the plant 202-1. The sample 122 can also include other information about the plant product of the sampled plant 202-1. For example, the sample 122 can include durometer and/or penetrometer measurements, volatile metabolites and other compounds that are detected non-destructively and/or destructively, etc.

In some implementations, the sample 122 can be extracted by one or more drones 120 or other types of collection devices or mechanisms. Example collection devices can include stationary probe devices and/or automated stationary samples. For example, a human can collect the sample 122 using one or more types of collection devices described herein. In some implementations, the sample 122 can be preprocessed to prepare the sample 122 for input to the nucleic acid sequencer 130 for sequencing. Preprocessing can include, for example, processing the sample 122 to obtain nucleic acid samples from the sample 122, barcode the sample, or any combination thereof. In some implementations, the sample 122 may include only the aerial portion of a plant such as plant 202-1. In such implementations, the aerial portion of the plant can be analyzed using the techniques of the present disclosure to determine a level of expression of one or more infection biomarkers in the nucleic acid derived from the plant 202-1's sample 122. Predictions can then be made as to whether the plant products (depicted in FIG. 1A using black dots on the plants, e.g., 102-1 through n02-z depicted in zone-1 through zone n, where n is any positive integer, x is any positive integer, y is any positive integer, where z is any positive integer) have or are likely to develop latent infections based on processing of only the aerial portion of the particular plant, e.g., plant 202-1. In some implementations, samples from both the aerial portion of the plant and the plant product can be used.

The nucleic acid sequencer 130 can process the received nucleic acid derived from the sample 122 and generate output data, which is referred to as read sequence data 132 that represents an order of nucleotides in the nucleic acid (e.g., DNA) of the sample 122. In some implementations, the read sequence data 132 can include the nucleotides in the nucleic acid of the sample 122 using one or more nucleotide bases that can include guanine (G), cytosine (C), adenine (A), and thymine (T) in any combination. In some implementations, the read sequence data 132 can be generated by transcribing read sequence data generated by the nucleic acid sequencer 130 into cDNA to represent the RNA of the sample 122. In such implementations, the read sequence data can be represented using bases that include G, C, A, and uracil (U) in any combination.

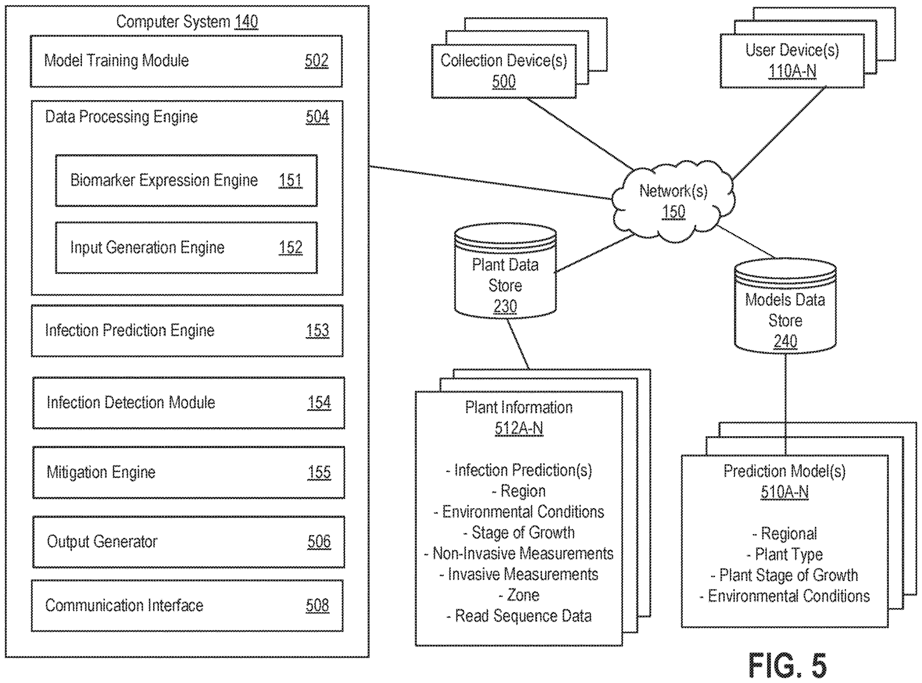

The read sequence data 132 can be provided to the computer 140 using the one or more networks 150. The one or more networks 150 can include a wired Ethernet network, an optical network, a wireless network, a WAN, a LAN, a cellular network, a Wi-Fi network, the Internet, or any combination thereof. In some implementations, the nucleic acid sequencer 130 can be directly connected to the application server using an Ethernet cable, a USB-C cable, or any other form of direct connection that facilitate communication of data between the nucleic acid sequencer 130 and the computer 140. The computer 140 can provide the read sequence data 132 as an input to a biomarker expression engine 151. The biomarker expression engine 151 can be part of the computer 140, as described further in reference to FIG. 5.

In some implementations, the data outputted by the nucleic acid sequencer 130 can be epigenetic data. The epigenetic data can include changes (e.g., chemical modifications, changes to the structure of the DNA, or the like) to the nucleic acid of the sample 122 that may not result in a change of base call (or nucleotide) of read sequence data 132 generated by the nucleic acid sequencer 130 based on the nucleic acid sequencer 130 sequencing of the sample 122. In some implementations, such epigenetic data can be generated by, for example, a long-read sequencer. In some implementations, if epigenetic data is not used, other nucleic acid sequencers can be used, including but not limited to short-read sequencer(s). While these are merely examples of sequencers that may be used to detect epigenetic data, any other sequencer can be used to generate the epigenetic data described herein.

The biomarker expression engine 151 can be configured to receive the read sequence data 132 and determine a level of expression of one or more infection biomarkers and optionally a level of housekeeping biomarkersas described in PREDICTION OF INFECTION IN PLANT PRODUCTS, International Publication Number WO 2021/092251 A1. The biomarker expression engine 151 can generate output data 151a based on processing the read sequence data 132, which represents the level of expression of the one or more infection biomarkers and optionally a level of housekeeping biomarkers. In some implementations, the level of expression of each of the biomarkers may include a number of occurrences of each gene in the read sequence data 132. The number of occurrences can be presented as a copy number.

In this example, a nucleic acid sequencing device 130 is used to sequence the sample 122 and generate read sequence data 132. Then, the biomarker expression engine 151 can determine the level of expression of one or more infection biomarkers and optionally one or more housekeeping biomarkers. In such implementations, this may include determining, by the biomarker expression engine 151, a number of occurrences of genes presented as the copy number. The present disclosure is not so limited and in some implementations, a nucleic acid sequencer 130 may not be required.

For example, in some implementations, a quantitative polymerase chain reaction device (qPCR) machine and microarray can be used to determine a number of occurrences of genes in the sample 122. In such implementations, the qPCR machine can be configured with primers to identify a particular gene sequences in the sample 122. The qPCR machine can be configured to amplify particular gene sequences identified by an assay of target genes. As a result, the qPCR machine can be faster than sequencing a complete genome or transcriptome using one or more types of sequencing devices, as the output of the qPCR is a count of only a subset of particular genes identified by the assay. The count output by the qPCR machine can be provided to the computer 140, the biomarker expression engine 151, and in some implementations, to an input generation engine 152 without first being provided to the biomarker expression engine 151. The qPCR machine can be implemented on the user device 110, the computer 140, or some other computer, device, and/or system that can be configured to communicate with the computer 140 using the network(s) 150.

The input generation engine 152 can be part of the computer 140 and can receive the generated output data 151a representing the level of expression of the one or more infection biomarkers and optionally level of housekeeping biomarkers, or both. The input generation engine 152 can generate an input data structure for input to the latent infection prediction engine 153 based on the received data 151a. In some implementations, this can include encoding data representing the level of expression of the infection biomarkers and optionally the housekeeping biomarkers, into an array data structure. For example, the encoded array data structure can include a string of binary digits representing the one or more infection biomarkers and optionally a level of housekeeping biomarkers, or both. The input generation engine 152 can provide the encoded input data structure 152a as an input to a latent infection prediction engine 153.

The latent infection prediction engine 153 can be part of the computer 140 and can receive the input data structure 152a. The latent infection prediction engine 153 can be or otherwise include one or more machine learning models that are trained using the training processes described in PREDICTION OF INFECTION IN PLANT PRODUCTS, International Publication Number WO 2021/092251 A1. The one or more machine learning models can generate output data based on processing the input data structure 152a that encodes data representing a level of expression of the one or more infection biomarkers and optionally the one or more housekeeping biomarkers, or both. The one or more machine learning models can also be provided with additional input data, including but not limited to destructive (e.g., invasive) measurements taken from the sampled plant 202-1, non-destructive (e.g., non-invasive) measurements taken from the sampled plant 202-1, data about environmental conditions (e.g., weather patterns, levels of precipitation, days of sunlight, other growing conditions), and/or data about a geographic region where the sampled plant 202-1 is grown and harvested. Using the additional input data and/or the input data structure 152a, the one or more machine learning models can predict likelihood of latent infection development in the sampled plant 202-1 and other similarly sourced plants within the zone-2 or other zones in a growing area (e.g., farm). The predictions can be in the form of output data.

The output data can therefore represent a likelihood 153a that the plant 202-1 from which the sample 122 was derived has, is developing, and/or may be developing one or more latent infections. The latent infection prediction engine 153 can perform the functionality described by the prediction models described in PREDICTION OF INFECTION IN PLANT PRODUCTS, International Publication Number WO 2021/092251 A1. The output data 153a generated by the latent infection prediction engine 153 can be provided to a latent infection detection module 154. In some implementations, the latent infection prediction engine 153 can include one or more of a binary logistic regression model, logistic model tree, random forest classifier, L2 regularization, or one or more neural networks, and/or a partial least squares model.

The latent infection detection module 154 can be part of the computer 140 and can evaluate the output data 153a to determine whether or not mitigation efforts may be required. By way of example, the output data 153a can be measured against one or more predetermined thresholds to determine whether the output data 153a indicates that the plant 202-1 from which the sample 122 was derived has, is developing, and/or may be developing one or more latent infections. In some implementations, the latent infection detection module 154 can evaluate the output data 153a in the same or similar manner as the output data of the prediction modules described in PREDICTION OF INFECTION IN PLANT PRODUCTS, International Publication Number WO 2021/092251 A1. If the latent infection detection module 154 determines that the output data 153a does not indicate presence of a latent infection, for example, then the techniques described herein can terminate at 156. If, alternatively, the latent infection detection module 154 determines that the output data 153a is indicative of the presence of a latent infection, then the latent infection detection module 154 can provide instructions 154a to a mitigation engine 155 to determine, suggest, and/or perform one or more mitigation operations.

The mitigation engine 155 can be part of the computer 140 and can initiate determinations, suggestions, and/or performance (e.g., execution) of one or more mitigation operations. These operations can occur in a variety of forms. By way of example, in some implementations, the mitigation engine 155 can determine, based on the instructions 154a, the output data 153a, the level of expression of one or more infection biomarkers and optionally the level of expression of one or more housekeeping biomarkers, that a harvest schedule for the sampled plant 202-1 and/or one or more other similarly sourced plants can be adjusted. In such implementations, the mitigation engine 155 can access a database storing a harvest schedule for the sampled plant 202-1, the plant products for the plant, similarly sourced plants and/or plant products, or a combination thereof, and adjust the harvest schedule based on the output data 153a. In such implementations, the output 155a of the mitigation engine 155 can include data written to the database to adjust the harvest schedule. In some implementations, adjusting the harvest schedule can include adjusting a post-harvest transportation route for the harvested plant products from the sampled plant 202-1 and/or similarly sourced plants. For example, plant products from the sampled plant 202-1 and/or other similarly sourced plants having a higher predicted likelihood of latent infection (e.g., predicted likelihood of latent infection exceeds some predetermined threshold value or range) can be scheduled for delivery to geographic regions that are closer to a harvesting location of the plant products in comparison to other plants having a lower predicted likelihood of latent infection or no latent infection, which can stay fresher longer and survive a further distribution. In some implementations, adjustments to the harvest schedule can be determined by the mitigation engine 155 and transmitted to the user device 110 or a computing device of another relevant stakeholder. The relevant stakeholder can then review the adjustments and determine whether to implement such adjustments. The adjustments can be implemented by the relevant stakeholder and/or semi-autonomously by the mitigation engine 155 or one or more other components described throughout this disclosure.

By way of example, in some implementations, the mitigation engine 155 can determine, based on the instruction 154a, the output data 153a, the level of expression of one or more infection biomarkers, and optionally the level of expression of one or more housekeeping biomarkers, that a plant, e.g., 202-1, a plant product, a zone of similarly sourced plants or plant products should be immediately harvested to avoid product-based waste. In such implementations, the mitigation engine 155 can generate an alert message that, when processed by a user device, can cause the user device to output an alert that notifies a user 105 of the user device 110 that the plant 202-1, plant product, and/or one or more other similarly sourced plants or plant products are to be harvested. In such instances, the output 155a can include the generated alert. Then, the computer 140 can transmit the generated alert to the user device 110.

The mitigation engine 155 can also determine one or more other actions that can be taken by the user 105, another relevant stakeholder, and/or another device/robot (e.g., the drone 120). For example, as described further below, the mitigation engine 155 can determine that one or more products can be sprayed on the plant 202-1, plant product, and/or one or more other similarly sourced plants or plant products to preserve such plant(s) and/or product(s) and prevent latent infection during the growing season before harvest.

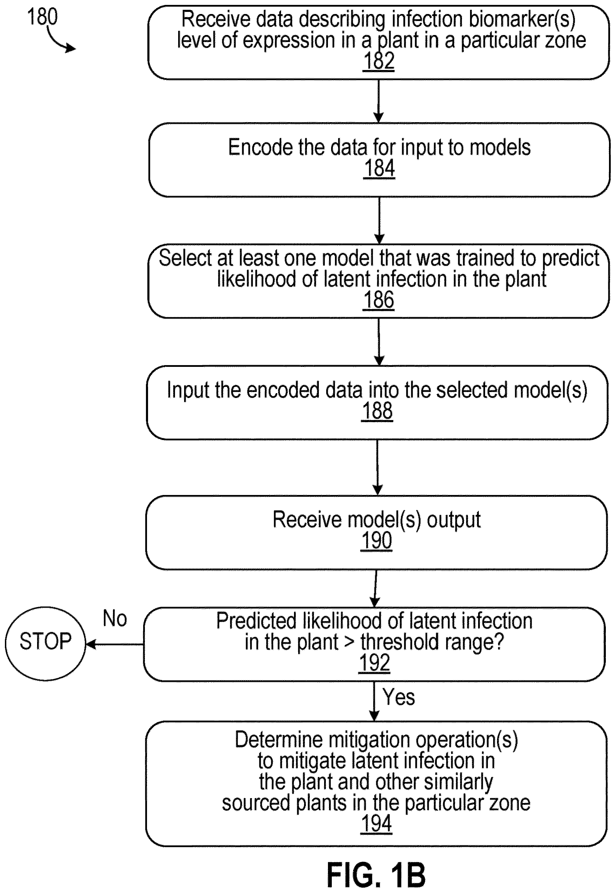

The mitigation engine 155 can also cause the plant 202-1 and other similarly sourced plants in a same zone to be harvested in a number of ways. For example, in some implementations, the mitigation engine 155 can cause deployment of one or more robotic devices (e.g., the drone 120) to begin harvesting plant products from the plant 202-1 and other locally-sourced plants within the same zone, such as zone-2 in FIG. 1. In such implementations, the mitigation engine 155 can generate one or more instructions that, when processed by a robotic device, cause the robotic device to (i) navigate to a location associated with the plant and (ii) initiate harvesting operations, by the robotic device. Initiation of harvesting by a robotic device can include causing the robotic device to begin performing one or more operations related to harvest of at least one plant product from the plant and one or more other similarly sourced plants in zone-2. In such implementations, the output 155a of the mitigation engine 155 can include instructions that cause the robotic device to navigate to and initiate harvest. In some implementations, the mitigation engine 155 can determine a location of the plant and the other similarly sourced plants in zone-2 using location data of the user device 110 of the user 105 or drone 120 that sampled the plant 202-1. The mitigation engine 155 can transmit, using the network 150, the one or more instructions to the robotic device 124, 125, and/or 126 and/or the robotic devices 120, or any other type of automated harvest collection device. Though depicted as quad-copter robotic devices, the present disclosure is not so limited. Instead, the robotic devices 124, 125, and/or 126 can include any robotic devices including robotic devices that navigate as rovers on the ground, robotic devices that navigate via flying through the air, or any combination thereof.

As yet another example, in some implementations, the mitigation engine 155 can determine, based on the instruction 154a, the output data 153a, the level of expression of one or more infection biomarkers and optionally the level of expression of one or more housekeeping biomarkers, that an antimicrobial treatment is to be prescribed for one or more other similarly sourced plants. In some implementations, antimicrobial treatments can be prescribed as described further below (e.g., refer to Table 1) and in PREDICTION OF INFECTION IN PLANT PRODUCTS, International Publication Number WO 2021/092251 A1. The mitigation engine 155 can cause administration of the antimicrobial treatment in a number of different ways.

For example, in some implementations, the mitigation engine 155 can generate one or more instructions that can be transmitted to a remote irrigation controller 171 using the network 150 to cause the irrigation controller 171 to activate the irrigation system 170 to deploy an antimicrobial treatment. For example, the irrigation controller 171 or the mitigation engine 155 can instruct the valve 172 to close and the valve 174 to open. The irrigation controller 171 can then spray the plant 202-1 and one or more locally sourced plants in the same zone such as zone-2 with the antimicrobial treatment to mitigate the detected or otherwise predicted latent infection. In other implementations, the mitigation engine 155 may determine based on the instruction 154a, the output data 153a, the level of expression of one or more infection biomarkers, the level of expression of one or more housekeeping biomarkers, or a combination thereof, that the plant 202-1 and other similarly sourced plants in zone-2 merely need watering and may not prescribe a treatment of antimicrobial solution. In such implementations, the mitigation engine 155 can instruct the irrigation controller 171 (e.g., the valves 174, 172) to be reconfigured to only disperse water from the water source 178, which may be a water tank, public water source, well water source, or a combination thereof.

The mitigation engine 155 can also cause antimicrobial solutions to be dispersed in other ways. For example, in some implementations, the mitigation engine 155 can instruct one or more robotic devices to initiate antimicrobial operations. In such implementations, a robotic device, such as the robotic device 120, may be equipped with a tank of antimicrobial solutions and a spraying device 121a, 122a respectively. In such implementations, the robotic device 120 can receive the instructions generated and transmitted by the mitigation engine 155, process the instructions, and navigate to a location associated with a plant and other similarity sourced plants in the same zone and use the sprayers 121a, 122a to administer the antimicrobial solution to the plant and other similarly sourced plants within the same zone.

The illustrative examples described above can analyze read sequences to determine gene counts and/or determined gene counts using a qPCR machine to detect one or more infection biomarkers, one or more housekeeping biomarkers, or both. The present disclosure is not so limited. Instead, the system 100 can be used to identify and count other types of biomarkers for input to the prediction engine 153. For example, in some implementations, the system 100 can be used to detect and count genes that may be indicative of drought stressors. An example of the types of genes that may be detected and counted as being indicative of drought stressors are described further below, such as in Table 1. The level of expression of these drought stressors can be analyzed using the system 100 in the same or similar manner as the level of expression of the infection biomarkers, the housekeeping biomarkers, or both. Detection, by the system 100, of a threshold level of expression of drought stressors can cause the system 100 to use the mitigation engine 155 to instruct the irrigation system 171 to perform watering of a particular plant and/or one or more similarly sourced plants within the same zone.FIG. 1B is a flowchart of a process 180 for detecting latent infection in plants. The process 180 can be performed by the computer system 140 depicted and described throughout this disclosure (e.g., refer to FIGs. 1A, 5). The process 180 and/or one or more blocks of the process 180 can be performed by other computing systems, devices, network of computers/devices, cloud-based systems, and/or cloud-based services. For illustrative purposes, the process 180 is described from the perspective of a computer system.

Referring to the process 180 in FIG. 1B, the computer system can receive data describing infection biomarker(s) level of expression in a plant in a particular zone (182). As described in reference to FIG. 1A and throughout this specification, the data can be samples of one or more plants that are collected by a user (e.g., human worker) in the field/farm. The samples can also be collected by devices, such as drones or other automated collection devices.

The computer system can encode the data for input to one or more models (184). The data can be encoded into a data structure for input to the one or more machine learning models. Refer to description in FIG. 1A about the biomarker expression engine 151 and the input generation engine 152.

The computer system can select at least one model that was trained to predict likelihood of latent infection in the plant in 186. As described further below, different models can be selected for application based on a type of the plant, a stage of growth of the plant, and/or one or more environmental conditions. The models can be layered in application such that they are applied in series. The models may also be applied in parallel.

In 188, the computer system can input the encoded data in the selected model(s). The computer system can then receive output from the model(s) in 190. The output can include a value indicating likelihood that the plant has or will have a latent infection. The value can be a numeric value (e.g., on a scale of 1 to 100, 1 being least likely to develop the latent infection and 100 being most likely to develop the latent infection). The value can also be a string value and/or a Boolean value.

In 192, the computer system can determine whether the predicted likelihood of latent infection in the plant (e.g., the output from the model(s)) is greater than a predetermined threshold range. If the predicted likelihood exceeds the predetermined threshold range, then the computer system can determine one or more mitigation operation(s) to mitigate latent infection in the plant and other similarly sourced plants in the particular zone (194). In other words, the computer system may determine that the plant has a high enough likelihood of developing latent infection that action should be taken early on during growth of the plant to avoid causing plant waste and/or loss of the plant or its plant product at harvesting time.

In some implementations, the computer system can provide one or more mitigation operations as suggestions to a user device of a relevant user. The user can then choose which mitigation operations to implement. The mitigation operations can be manually performed by the user or other users. The mitigation operations may also be performed automatically by the computer system. The mitigation operations may also be performed semi autonomously by the computer system once the user provides input to the computer system indicating selection of such mitigation operations.

If the predicted likelihood of latent infection in the plant is not greater than the predetermined threshold range, then the process 180 can end. In other words, the computer system may determine that the particular plant does not have a high enough likelihood of developing latent infection to invoke some type of mitigation operation(s) at a present time during the plant's growth period.

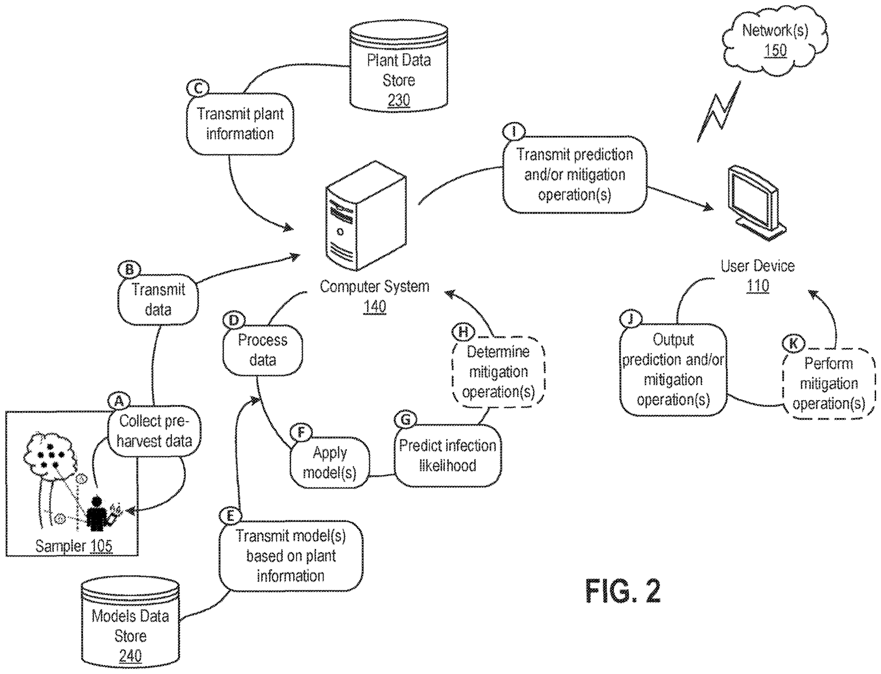

FIG. 2 is a conceptual diagram for predicting likelihood of infection in plants. The computer system 140, user device 110, plant data store 230, and models data store 240 can be in communication (e.g., wired, wireless) via the network(s) 150. A sampler 105 can collect pre-harvest data from a plant in a zone of a field/farm in step A. Pre-harvest collection can occur as early as 6 months before harvest. Pre-harvest collection may also determine at one or more time intervals during the growing season of the plant. For example, pre-harvest collection and subsequent predicting the likelihood of infection can occur every 2 months until harvest. As another example, pre-harvest collection and predicting the likelihood of infection can be performed 6 months before harvest. At this point, mitigation operations can be determined and applied to the plant and/or similarly sourced plants. It can also be determined at this point whether additional pre-harvest collection and/or prediction may be needed during the rest of the growth season before harvest.

Pre-harvest collection can include any of the techniques described herein (e.g., refer to FIG. 1A). For example, the sampler 105 (e.g., a user) can walk around the plant and collect a subset of plant products from the plant. In some implementations, the sampler 105 can remove 4 plant products from a plant that is being sampled and tested for likelihood of latent infection. The sampler 105 can remove any other quantity of plant products from the plant that may be optimal for predicting likelihood of latent infection. The quantity removed can depend on the type of plant, a particular region where the plant is grown, and/or one or more other environmental conditions. Collection of the plant products can therefore be used to determine whether a particular plant (e.g., a tree) in a particular zone in the field/farm may be infected.

As another example, collection can include detecting volatile metabolites of the plant using nondestructive techniques. Such data can also provide insight into how the plant responds to environmental conditions, which can be another indicator of whether the plant has latent infection and/or is likely to develop latent infection. The sampler 105 can hold a volatiles collection device (e.g., NIR device, electronic nose or other instrument) near the plant products to collect volatiles that are off-gassed. Other field-deployable instruments can also be used to collect and measure volatile components that are off-gassed by the plant products, including but not limited to electronic noses and/or NIR devices on semi-autonomous or autonomous robotic devices.

Any combination of pre-harvest collection can be performed. The pre-harvest collection may also depend on what model(s) is used to predict likelihood of latent infection in the plant product, which may further depend on the type of the plant, the growing region, and/or environmental conditions. For example, in some implementations, volatiles can be collected for plant products of the plant and a quantity of the plant products can also be removed from the plant and brought to a lab or facility for further analysis.

Once pre-harvest data is collected (step A), the data can be transmitted to the computer system 140 (step B). As described herein, the data can include sequencing data, which can be transmitted to a nucleic acid sequencer or other sequencing device (e.g., refer to FIG. 1A). The data may also include volatile measurements. In some implementations, the data may include other measurements that are obtained nondestructively and/or destructively of the plant products that are collected and/or removed from the plant.

The computer system 140 can receive plant information from the plant data store 230 (step C). The information can be specific about the type of plant that is being collected and tested for infection. The information can also be specific about a growing region of the collected plant and/or plant product. The information may also include current environmental conditions and/or future environmental conditions that may arise during the growth season of the plant, before harvest. Step C can be performed any time before, during, and/or after one or more steps described in FIG. 2. For example, step C can be performed at a same time as performing step D.

The computer system 140 can process the data in step D. Processing the data can be performed as described in FIG. 1A. Processing the data can include preparing the collected pre-harvest data by encoding it to be provided as input to one or more models. Processing the data can also include preparing the plant information for input to one or more models.

The computer system can also receive model(s) from the models data store 240 based on the plant information (step E). The computer system can determine how many models to apply to predict likelihood of infection in the plant. The computer system can also determine which model or models to select based on the plant information, such as the type of the plant and/or the environmental conditions.

The computer system can apply the selected model(s) in step F. For example, the computer system can provide the collected pre-harvest data and/or the plant information to the model as input (e.g., in encoded structures).

Accordingly, the computer system can predict likelihood of infection in the plant in step G. For example, the model(s) can provide output in the form of a value indicating likelihood of infection in the plant. The computer system can then determine whether the value exceeds some predetermined threshold range. Exceeding the threshold range can indicate that the plant has a high likelihood of developing infection in the plant. If the value does not exceed the threshold range, then the plant has a lower likelihood of developing infection.

Based on the prediction in step G, the computer system 140 can optionally determine mitigation operation(s) in step H. For example, refer to Table 1 below for additional discussion on types of mitigation operations that can be determined, suggested, and/or implemented (e.g., manually, autonomously, and/or semi-autonomously).

The computer system 140 can transmit the prediction of infection likelihood and/or determined mitigation operation(s) to the user device 110 (step I). The user device 110 can output the prediction and/or mitigation operation(s), to be presented to a relevant user (step J). For example, the user device 110 can be a mobile phone, computer, laptop, and/or tablet having a display screen. The prediction and/or mitigation operation(s) can be presented on the display screen in a graphical user interface (GUI). The relevant user can view such information and perform actions in response to viewing the information. For example, the relevant user can select one or more of the presented mitigation operation(s) to be executed.

The user device 110 and/or the computer system 140 then optionally perform the mitigation operation(s) (step K). In some implementations, the relevant user can select mitigation operations to be performed at the user device 110. A notification of this selection can be transmitted back to the computer system 140. The computer system can then send a notification and instructions to one or more robotic devices to perform the user-selected mitigation operations. In some implementations, the user device 110 can transmit instructions to the one or more robotic devices when mitigation operations should be performed. Sometimes, instructions can be transmitted to one or more other user devices, notifying users of such devices that they should perform one or more mitigation operations. Yet in some implementations, the user device 110 and/or the computer system 140 may perform the mitigation operation(s) in step K without receiving selection and/or approval from the relevant user at the user device 110.

**Table 1: Information or alerts that can be obtained using a combination of pre-harvest biomarker values from one or more of the following gene categories (with example genes) and potential resultant actions based on these values.**

| **Alert/Information** | **Applicable Gene Categories** | **Specific Examples** | **Potential Resultant Actions** |
|---|---|---|---|
| Infection likelihood of product after harvest; prediction of product quality post-harvest | Phenylpropanoid pathway; General stress response; Defense response to pathogen; Ethylene synthesis; Ethylene signaling; ABA synthesis; ABA signaling; Jasmonic acid synthesis; Jasmonic acid signaling; Salicylic acid synthesis; ERF/AP2 transcription factors; Auxin synthesis Auxin transport | PAL, CHS; PER, HSPs Chitinases, WRKYs, NACs; ACO, ACS; ERFs, ETRs, EINs; NCED, ZEP; ABI4; JMT, LOX; JAZ; SAMT; EIN3; YUCCA; PIN proteins; | (1) Alert farm manager to harvest tree/crop or section of trees/crops early in season or in dry weather conditions; |
| | | | (2) Inform farm manager of how to handle product postharvest (suggested temperature in transit, suggested maximum time in transit, etc.); |
| | | | (3) Automatically alert packing house where product is being sent as a "quality prediction score" that can inform which market the product is being sent to (high end retail, local market, juicing/processing, etc.), what types of postharvest treatments or coatings should be applied (suggest application or no application of pesticide, wax or other shelf life extension technology, application of ripening agent or induction/triggering of ripening); |
| | | | (4) Alert farm manager to apply a specific type or amount of pesticide to a single tree/crop or set of trees/crops; |
| | | | (5) Alert drone to automatically apply a specific type or amount of pesticide to a single tree/crop or set of trees/corps; |
| Tree/crop age or replant time prediction | | | (1) Alert farm manager when a tree is nearing the end of its healthy production lifespan; |
| | | | (2) Predict years of production from a tree or crop |
| Prediction of bud wood health | | | (1) Enable farm manager to make informed decision as to which parent trees would produce the best (healthy, uninfected) bud wood for grafting |
| Water stress detection/estimation | | | (1) Alert farm manager of water stressed trees/crops |
| | | | (2) Send signal to irrigation system to automatically increase or decrease irrigation to a tree/crop or section of trees/crops |
| | | | (3) Send signal to drone to automatically increase or decrease irrigation to a tree/crop or section of trees/crops |
| | | | (4) Enable optimization of water stress to produce heartier crop without over-stressing parent plant by providing real-time analysis of stress status, which could be used by the farm manager to manually alter irrigation or automatically trigger changes in irrigation through signals to system or drone |
| Nutrient stress estimation | | | (1) Alert farm manager of nutrient stressed trees/crops |
| | | | (2) Send signal to irrigation system to automatically increase or decrease addition of nutrient supplement via spray or soil additive to a tree/crop or section of trees/crops |
| | | | (3) Send signal to drone to automatically increase or decrease addition of nutrient supplement via spray or soil additive to a tree/crop or section of trees/crops |
| | | | (4) Enable optimization of nutrient composition to produce heartier crop by providing real-time analysis of nutrient status, which could be used by the farm manager to manually alter nutrient application or automatically trigger changes in application through irrigation through signals to system or drone |
| Detect young tree/crop health or stress level; predict young tree/crop survival rate based on health/stress level | | | (1) Inform farm or nursery manager of the health, stress, and/or disease status of a young tree or crop such that these young trees can be prioritized or sold at a premium; |
| | | | (2) Inform farm or nursery manager of the health, stress, and/or disease status of a young tree or crop such that amendments can be made to the soil, irrigation levels, or environment (relative humidity, temperature) of the trees/crops to reduce stress; |
| | | | (3) Inform drone or automation system of the health, stress, and/or disease status of a young tree or crop such that amendments can be automatically made to the soil, irrigation levels, or environment (relative humidity, temperature of a greenhouse) of the trees/crops to reduce stress; |
| | | | (4) Inform manager or machine of tree/crop stress such that transit conditions (temperature, relative humidity, etc.) can be manually or automatically changed to reduce stress and increase young tree/crop survival; |
| | | | (5) Inform manager or machine of tree/crop infection status or infection likelihood such that pesticide application can be manually or automatically prescribed and implemented to mitigate pathogenesis and increase young tree/crop survival |
| Temperature or sun exposure stress detection/ prediction | | | (1) Alert farm manager of water stressed trees/crops; |
| | | | (2) Send signal to irrigation system to automatically increase or decrease irrigation to a tree/crop or section of trees/crops; |
| | | | (3) Send signal to drone to automatically increase or decrease irrigation to a tree/crop or section of trees/crops; |
| | | | (4) Send signal to drone to automatically apply sunscreen or heath mitigation product to a tree/crop or section of trees/crops; |
| | | | (5) Enable optimization of application of sunscreen and/or heath mitigation products through real-time analysis of heat and sun stress |

The example gene categories in the Table 1 include PAL: phenylpropanoid ammonia lyase; CHS: chalcone synthase; PER: peroxidase; HSPs: heat shock proteins; WRKYs: WRKY transcription factors; NACs: NAC transcription factors; ERFs: ethylene response factor; ETRs: ethylene transcription factor; EINs: ethylene insensitive protein; ABA: abscisic acid; NCED: 9-cis-epoxycarotenoid dioxygense; ZEP: zeaxanthin epoxidase; ABI: abscisic acid insensitive transcription factors; JMT: jasmonic acid methyltransferase; SAMT: salicylic acid methyltransferase; and PIN: PIN-FORMED proteins. One or more other gene categories can also be used with the disclosed techniques.

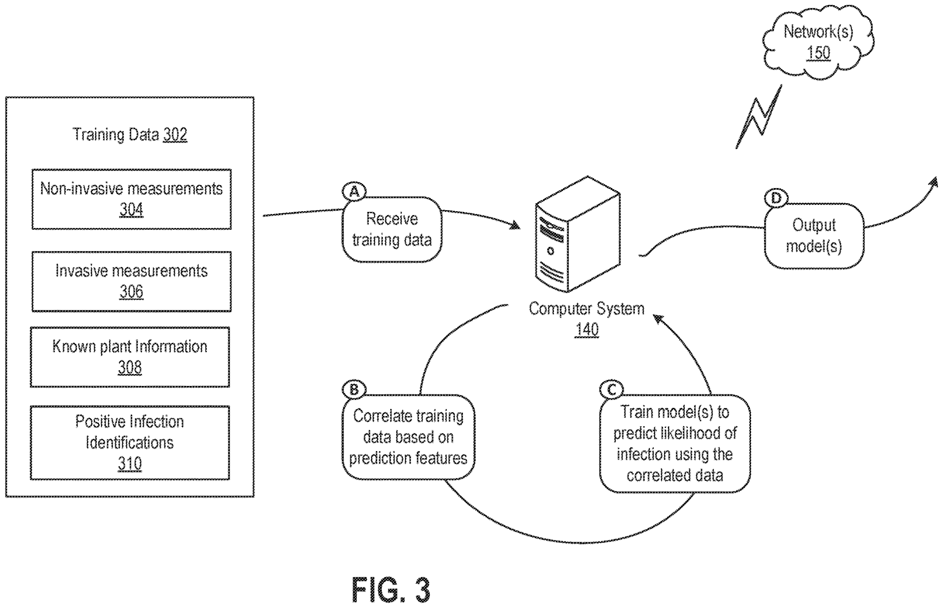

FIG. 3 is a conceptual diagram for training one or more models for predicting the likelihood of infection in plants. The models can be trained using any of a variety of techniques, for example, including any of the techniques described in this document and any of the techniques described in PREDICTION OF INFECTION IN PLANT PRODUCTS, International Publication Number WO 2021/092251 A1, which is filed with one or more of the same inventors.

Although training of the one or more models is shown as performed by the computer system 140, training can also be performed by one or more other computer systems, devices, networks, cloud-based systems, and/or cloud-based services.

Referring to FIG. 3, the computer system 140 can receive training data 302 from one or more source (step A). The training data 302 (or portions thereof) can be received from a user device of a relevant user, a volatiles collection device, a robotic device, an NIR device, one or more other computing devices, and/or one or more data stores. In some implementations, the computer system 140 can select one or more types of the training data 302 to receive.

The training data 302 includes one or more of non-invasive measurements 304, invasive measurements 306, known plant information 308, and positive infection identifications 310. The non-invasive measurements 304 can be collected with devices such as NIR devices, electronic noses, and other volatiles collection devices. The non-invasive measurements 304 can include, for example, data indicating volatiles composition of plants as they are out-gassed by the plants. The non-invasive measurements 304 can be taken of plant products that are on plants in a field (e.g., farm). Thus, the plant products may not be removed from the plants. In some implementations, the non-invasive measurements 304 can be taken of the plant products when they are removed/picked from the plants and used for further analysis in a lab or other analysis setting. The non-invasive measurements 304 can also include nucleic acid samples of the plant products that are being analyzed.

The invasive measurements 306 can be collected with devices such as penetrometers and durometers. The invasive measurements 306 can be collected, by a relevant user, of plant products that are still part of the plants and/or plant products that are removed from the plants and used for further analysis. For example, the relevant user can remove a quantity of plant products (e.g., fruits) from a plant (e.g., tree) and bring the quantity of plant products back to a lab or other analysis setting. In the lab, the relevant user can capture measurements of the plant products by, for example, squeezing the plant products to identify firmness measurements, removing some portion of the skin of the plant products, measuring a thickness of the skin by puncturing the plant products, etc. The invasive measurements can be used to determine percent dry matter, fatty acid, sugar content/profiling, and/or acid content/profiling. Moreover, such information can be determined using various other types of analytical methods, including but not limited to volatile analysis and metabolomics.

The known plant information 308 can include historic growing information about particular plants, such as a length of a growing season and environmental conditions that support growth, maturation, and/or ripening of the particular plants. The environmental conditions can include levels of precipitation, amounts of sunlight, and/or preferred temperatures for growing, maturing, and ripening of the particular plants.

The positive infection identifications 310 can indicate instances when particular plants have been identified as having latent infection or otherwise developing latent infection. For example, the identifications 310 can indicate what levels or expressions of biomarkers have been positively associated with one or more types of latent infection for a particular plant. These associations can be made by a relevant user. These associations can also be made by a computer, such as the computer system 140. The positive infection identifications 310 can also indicate combinations of levels or expressions of biomarkers, environmental conditions, and one or more other information about the plant that have been positively associated with one or more types of latent infection for a particular plant.

Once the computer system 140 receives the training data (step A), the computer system 140 can correlate the training data based on prediction features (step B). In other words, the computer system 140 can make associations between the non-invasive measurements 304, invasive measurements 306, known plant information 308, and positive infection identifications 310. The associations can indicate likelihood of latent infection in a particular plant. For example, a combination of a particular volatile off-gassed by the particular plant product and abnormal precipitation patterns and temperature during the growing season can indicate development of latent infection in the particular plant and similarly sourced plants during the growing season, before harvest. This combination of information can be correlated with a positive infection identification 310 of the particular plant.

As mentioned, the training data can be correlated based on prediction features. The prediction features can include but are not limited to plant type, growth region, stage of growth, ripening conditions, stage of ripening, environmental conditions etc. The prediction features can therefore represent different characteristics during the growth, maturation, and/or ripening period of a particular plant and similarly sourced plants that may affect development of latent infection. In some implementations, models can be generated for each of the prediction features. For example, one model can be generated that predicts latent infection in avocados based on where the avocado is grown. As another example, another model can be generated that predicts latent infection (e.g., a particular type of latent infection, such as SER, or any type of latent infection) in a particular type of avocados based on volatiles that are off-gassed by such avocados during the growth stages. Another model can also be generated that predicts latent infection in a particular type of produce based on timing pre-harvest at which the plant's gene expression is collected. For example, one model can be used for predicting latent infection in avocados during 0-3 months of growth and another model can be used for predicting latent infection in avocados during 3-6 months since start of growth. This can be beneficial since gene expression can change based on age of the plant product and therefore can provide different indications of latent infection based on whether the gene expressions are collected and analyzed during 0-3 months of growth versus 3-6 months of growth. One or more other time intervals can be used in generating models.

In some implementations, one or more of the prediction features can be combined to generate a model. For example, one model can be generated that predicts latent infection in avocados based on a combination of volatiles off-gassed by the avocados and environmental conditions. As another example, another model can be generated that predicts latent infection in limes based on a combination of environmental conditions (e.g., temperature, precipitation), stage of growth of such limes, and region of growth. One or more other combinations of prediction features are also possible. As yet another example, a model can be generated that predicts latent infection in different types of plants based on the respective plant type, region of growth, off-gassed volatiles, and environmental conditions. The same model can therefore be used to predict latent infection in different plants, including but not limited to avocados, limes, lemons, apples, citrus fruits, etc.

In yet some implementations, models can be generated per growing region. Then additional models can be generated per region based on age, growth stage, environmental conditions, and other information. Thus, models can be developed within models in order to provide for more accurate prediction of latent infection in plants early on before harvesting time. Therefore, mitigation operations can be determined and implemented early enough pre-harvest to reduce or otherwise eliminate plant-based waste come harvesting time.

The computer system 140 can train the model(s) to predict likelihood of infection using the correlated data (step C). For example, the correlated data can be provided as input to the model(s). The model(s) can generate output as a value indicating likelihood of infection in a particular plant associated with the inputted, correlated data. The computer system 140 can compare the output of the model(s) to the positive infection identifications 310 to further refine and improve accuracy of prediction determinations made by the model(s). Once a desired level of accuracy is reached, the computer system 140 can output the model(s) (step D). The computer system 140 can also continuously improve the model(s) in a feedback loop, as the model(s) are deployed and used during runtime.

Overall, some biomarkers in plant products can dictate what models to generate and/or use during runtime. Biomarkers collected at different ages over different growing seasons can be used to verify accuracy of the models in predicting likelihood of latent infection development pre-harvest time. Invasive and/or non-invasive collection of hormones, gene expressions, dry matter (e.g., oil content, maturity), etc. can also be used to determine what models to generate and/or use during runtime.

The model(s) can be trained to generate output that indicates a likelihood of whether a particular plant and/or similarly sourced plants will develop or currently is developing a particular type of latent infection or any type of latent infection. In some implementations, the model(s) may also be trained to determine one or more mitigation operations that can be taken by relevant users to prevent development of the predicted latent infection. For example, the model(s) can be trained to select one or more mitigation operations from a repository of known mitigation operations typically performed by the relevant users. The model(s) can be trained to select optimal mitigation operations to reduce or prevent plant-based waste at harvesting time. This can save time of the relevant users because then the relevant users do not have to think about what type of mitigation operations may be preferred in the particular situation. Instead, the relevant user can review the suggested mitigation operations, select one or more, and manually or semi-autonomously implement such mitigation operations.

In some implementations, the model(s) can be built using hierarchical cluster analysis based on classification. For example k-means clustering techniques can be used to build and train the model(s). Clustering analysis can be beneficial since gene expressions may trend similarly and therefore provide clearer clusters for analysis and prediction determinations. Thus, the models can more accurately predict infection development. One or more other techniques can also be used to build and/or train the model(s), including but not limited to random forest modeling. Decision tress may also be used to correlate/associate gene expression data, plant information, and other invasive and/or non-invasive plant measurements/data. Thus, any of the techniques described herein can be used to train the model(s) to correlate non-invasive measurements 304 with invasive measurements 306 to determine pre-harvest development of infection.

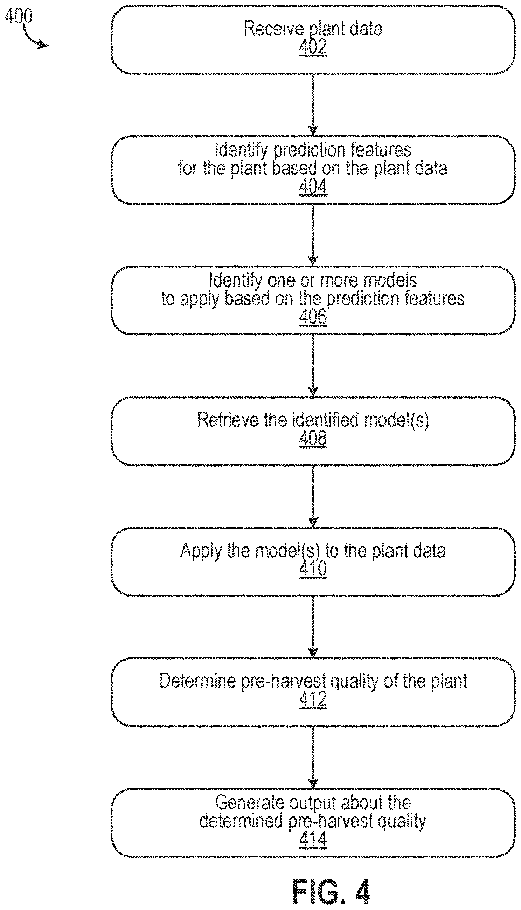

FIG. 4 is a flowchart of a process 400 for predicting likelihood of infection in plants. The process 400 can be used to predict development of latent infection in a particular plant and/or similarly sourced plants. Such prediction can be made pre-harvest, sometimes 6 months before harvesting time. The process 400 can also be performed at any other time before harvesting the plant and/or similarly sourced plants such that mitigation operations can be performed sufficiently before harvest to reduce or prevent plant-based waste.

The process 400 can be performed by the computer system 140 depicted and described throughout this disclosure (e.g., refer to FIGs. 1A, 5). The process 400 and/or one or more blocks of the process 400 can be performed by other computing systems, devices, network of computers/devices, cloud-based systems, and/or cloud-based services. For illustrative purposes, the process 400 is described from the perspective of a computer system.

Referring to the process 400 in FIG. 4, the computer system can receive plant data in 402. Refer to FIGs. 1-2 for further discussion about receiving the plant data. In 404, the computer system can identify prediction features for the plant based on the plant data. In other words, analysis of the plant data may lead to identification of one or more features that can be used to select appropriate models to predict likelihood of infection in the corresponding plant. The prediction features can include but are not limited to plant type, stage of growth, geographic region/location, soil type, age, hormones, gene expressions, dry matter, and environmental conditions. As described herein, different prediction models can be used for each plant type, stage of growth, geographic region/location, soil type, age, hormones, gene expressions, dry matter, and/or environmental conditions. By identifying the prediction features based on the plant data, the computer system can therefore determine how many and which prediction models would be appropriate to predict latent infection in the corresponding plant. Sometimes, a geographic region/location feature identified from the plant data can be used to select a subset of models that are trained to predict latent infection in plants in that geographic region/location. Environmental conditions or one or more other prediction features that are also identified based on the plant data can then be used to select one or more models from the subset of models selected based on the geographic region/location feature. Accordingly, the computer system can identify one or more models to apply based on the prediction features in 406.

In 408, the computer system can retrieve the identified model(s). The computer system can then apply the model(s) to the plant data in 410. The plant data can be provided as input to the model(s). One or more different portions of the plant data can be inputted into each of the model(s). One model, as an illustrative example, can receive environmental conditions data as input to predict likelihood of latent infection and/or likelihood of a particular type of latent infection. Another model can receive plant gene expressions, volatile components, and/or firmness measurements from the plant data as input to predict likelihood of latent infection and/or likelihood of a particular type of latent infection. Yet another model can receive a combination of different inputs, such as environmental conditions and gene expressions to predict likelihood of latent infection in the plant. The models can be trained to receive one or more other inputs and/or combinations of inputs.

The computer system can determine pre-harvest quality of the plant based on application of the model(s) to the plant data in 412. For example, the computer system can determine whether output from the one or more models, alone and/or in combination, exceed some predetermined threshold range or value. If the output exceeds the predetermined threshold range or value, the computer system can determine that the plant is going to develop latent infection and/or will be developing latent infection. On the other hand, if the output does not exceed the predetermined threshold range or value, the computer system can determine that the plant does not have any latent infections and/or is not likely to develop infection based on current conditions of the plant and/or environment.

As another example, the computer system can average the outputs from the applied models and determine whether the averaged output exceeds the predetermined threshold range or value. The computer system can also identify a mean output of the models. In some implementations, the computer system may perform a majority vote ruling and identify output that is similarly expressed for a majority of the applied models. The computer system can then adopt that output as the pre-harvest quality of the plant in 412. Moreover, in 412, the computer system can determine the pre-harvest quality of similarly sourced plants. The similarly sourced plants can be part of a same tree, zone, and/or area of a farm or other growing region as the plant whose plant data is received in 402. In some implementations, the similarly sourced plants can also be located in another growing region but may experience similar environmental conditions, soil, and/or other prediction features as the plant whose plant data is received in 402.

In 414, the computer system can generate output about the determined pre-harvest quality of the plant. The computer system may also store the determined pre-harvest quality of the plant. The computer system can transmit the output to a user device to be presented to a relevant user, such as a farmer or other user in the supply chain. The output can indicate the pre-harvest quality of the plant and/or likely quality of the plant until the plant is harvested. The output can also indicate pre-harvest quality of similarly sourced plants. The computer system may also suggest one or more operations that can be performed to mitigate development of latent infection pre-harvest. Optionally, the computer system may automatically execute one or more mitigation operations. The suggested and/or executed mitigation operations can include any of the operations listed in Table 1 above. One or more other mitigation operations can also be suggested and/or executed.

FIG. 5 is a system diagram of one or more components that can be used to perform the techniques described herein. As described herein, the computer system 140, user device(s) 110A-N, plant data store 230, and models data store 240 can communicate via the network(s) 150. Collection device(s) 500 can also communicate with the components described herein via the network(s) 150. The collection device(s) 500 can include any of the collection devices described herein, particularly in reference to FIG. 1A, including but not limited to handheld samplers of a user 105, sampler robotic devices 120, harvester robotic devices 160, 162, 164, electronic noses/sniffers, and/or other automated robotic devices that can collect samples from plants and plant products.

The computer system 140 can include a model training module 502, a data processing engine 504, the latent infection prediction engine 153, the latent infection detection module 154, the mitigation engine 155, an output generator 506, and a communication interface 508. The computer system 140 can optionally include one or more additional, fewer, or different components that can be used to perform the techniques described throughout this disclosure.

The model training engine 502 can be configured to train prediction model(s) 510AN. The engine 502 can receive training data, which can be stored in the plant data store 230 and/or the models data store 240. The engine 502 can use the training data to train the prediction model(s) 510A-N to predict latent infection development in plants pre-harvest. The engine 502 can train prediction models 510A-N that are based on growing region, plant type, plant stage of growth, and/or environmental conditions. The engine 502 can also train one or more additional, fewer or different prediction models 510A-N, as described throughout this disclosure. Moreover, the engine 502 can train models within models. The engine 502 can also continuously train and/or validate the prediction models 510A-N using prediction determinations made during runtime. Models that are generated and trained by the engine 502 can be stored in the models data store 240 as the prediction models 510A-N.

The data processing engine 504 can be configured to process plant data that is received from the collection devices 500, as described throughout this disclosure. In some implementations, the plant data collected by the collection devices 500 can be stored as plant information 512A-N in the plant data store 230. The engine 504 can then retrieve the plant information 512A-N from the plant data store 230 and process that information as described herein. The plant information 512A-N can include but is not limited to infection prediction(s), region of growth, environmental conditions, stage of group, non-invasive measurements, invasive measurements, zone of growth in a field, farm, or other growing location, and/or read sequence data.

The biomarker expression engine 151 can be configured to perform the techniques described in FIG. 1A. The input generation engine 152 can also be configured to perform the techniques described in FIG. 1A.

The latent infection prediction engine 153 can be configured to predict a likelihood that the plant, and/or similarly sourced plants, are developing, developed, and/or are likely to develop latent infection pre-harvest. Refer to FIG. 1A for further discussion about the latent infection prediction engine 153. In brief, the latent infection prediction engine 153 can receive the plant data from the collection device(s) 500, the plant data store 230, and/or the data processing engine 504. Using the plant data, the latent infection prediction engine 153 can select one or more of the prediction models 510A-N from the models data store 240 to apply to the plant data. Based on applying the selected models 510A-N, the latent infection prediction engine 153 can predict likelihood that the plant is developing, may develop, and/or has developed latent infection(s) pre-harvest. The latent infection prediction engine 153 can store this prediction in the plant data store 230 as part of the plant information 512A-N.

The latent infection detection module 154, as described in reference to FIG. 1A, can be configured to determine one or more mitigation efforts that can be performed in response to a predicted likelihood of latent infection pre-harvest. Refer to FIG. 1A for further discussion about the latent infection detection module 154.

The mitigation engine 155 can be configured to determine whether one or more operations can and/or should be performed to mitigate potential development of latent infection in the plant and/or similarly sourced plants before harvest. The engine 155 can also determine one or more suggestions for mitigation operations to perform. The engine 155 can receive the prediction from the latent infection prediction engine 153 (or retrieve the prediction from the plant information 512A-N in the plant data store 230) to determine one or more mitigation operations. In some implementations, the mitigation engine 155 may optionally execute one or more of the mitigation operations with or without approval from a user at the user device(s) 110A-N. For example, in situations where the plant and/or similarly sourced plants has already developed latent infection and is likely to continue developing the infection to the point of plant-based waste by the time of harvest, the mitigation engine 155 can automatically implement one or more mitigation operations to try and reduce continued development of the infection.

The output generator 506 can be configured to generate output for presentation at the user devices 110A-N. The output generator 506 can receive the prediction determination(s) from the latent infection prediction engine 153 and/or mitigation operations from the mitigation engine 155. Using this information, the output generator 506 can generate output, such as messages, notifications, alerts, and/or alarms. The generated output can, for example, include a value indicating the predicted likelihood of latent infection in the plant and/or similarly sourced plants. The generated output can also include one or more of the mitigation operations that can be selected and performed (e.g., manually and/or automatically). The output can also include one or more other messages, notifications, alerts, and/or alarms as described herein. The output generator 506 can transmit the output to the user devices 110A-N for presentation to the user(s). The output generator 506 can also transmit the output to the plant data store 230 to be stored in the plant information 512A-N.

Finally, the communication interface 508 can provide communication between one or more of the components described herein.

The user devices 110A-N can include any one or more of computers, laptops, tablets, mobile devices, smartphones, cellphones, etc. that may be used by relevant users. The user devices 110A-N can include input device(s), output device(s), processor(s), and communication interfaces. Such components can allow the user to provide information to the computer system 140, view and/or access information from one or more of the components described herein, and/or take action based on information provided herein.

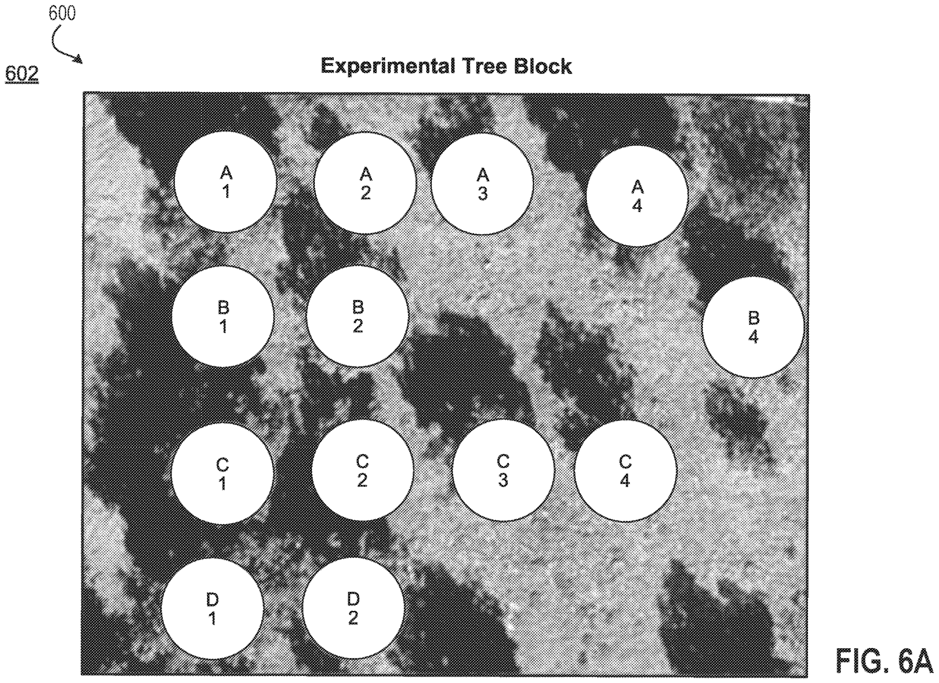

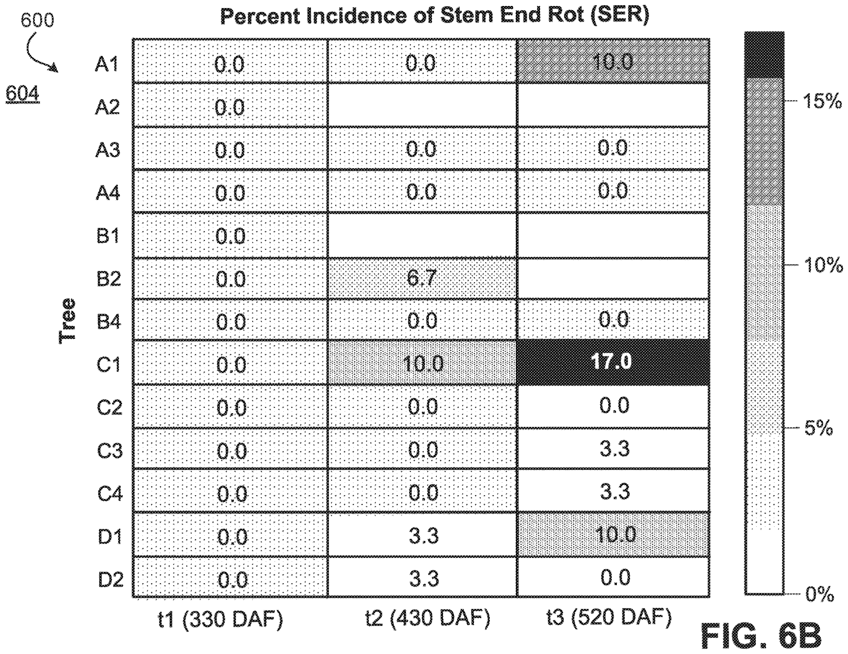

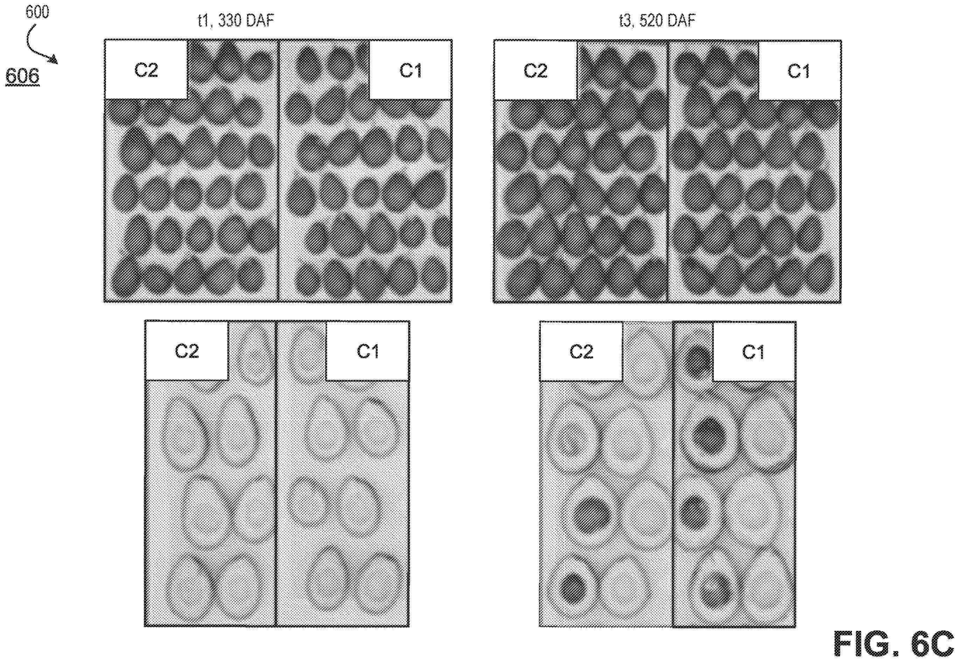

FIGs. 6A-C depict an example process 600 for collecting data of plants to use with the disclosed techniques. FIGs. 6-10 provide an illustrative example of how the disclosed techniques can be applied to a type of plant. FIGs. 6-10 are merely illustrative and do not limit application of the disclosed techniques to other types of plants, including but not limited to other perennial fruiting trees (e.g., mango, citrus, pear, apple, and stone fruit trees), vines (e.g., grape vines), and bushes (e.g., blackberry, raspberry, and/or strawberry bushes).

Referring to FIGs. 6-10, an avocado tree *(Persea americana)* can be a long-living perennial plant that can produce its highly desirable fruit crop at large volumes for decades. Throughout the lifespan of the avocado tree, it can constantly interact with and respond to populations of microbes. While many of these microbial relationships may be commensal or mutual, there also exists many examples of parasitism of the avocado tree and/or fruit that can cause severe losses in tree health, fruit yield, and fruit quality. Stem end rot (SER) can be a common quality defect of avocado fruit caused by parasitic invasion of flesh of the avocado fruit near the stem end as the fruit ripens post-harvest. The fungi that cause this disease may live biotrophically in the pedicel of the fruit prior to harvest and ripening typically without causing disease symptoms to the fruit or tree.

The disclosed techniques can therefore be used to correlate physical and molecular attributes of early, mid, and late season avocado fruit to a low or high incidence of SER upon harvest and ripening. Using RNA-seq, expression profiles can be generated, which may illustrate that, at all timepoints prior to peak harvest maturity, fruit from trees of high SER incidence can be recognizing and responding to increased fungal pressure through an increased expression of genes involved in canonical pathogen response pathways. Leveraging this information towards the production of detection assays can enable prediction of high and low incidence trees with ample time to make amendments to fungicide applications, fruit harvest schedule, or fruit market destination to mitigate manifestation of SER or other latent infections in the avocados.

In the process 600, 602 is an aerial view of avocado trees that can be sampled (e.g., refer to FIG. 6A). Heatmap 604 represents average SER incidence for each tree at each collection timepoint (e.g., refer to FIG. 6B). The incidence values for each tree at each timepoint are illustrated in each box in the heatmap 604. Moreover, blank boxes indicate that a particular tree was unavailable for sampling at the timepoint due to a low volume of fruit or ongoing tree management/pruning. Image data 606 represent differences between low (C2) and high (C1) incidence trees at t1 (330 days after flowering, DAF) and t3 (520 DAF) (e.g., Refer to FIG. 6C).

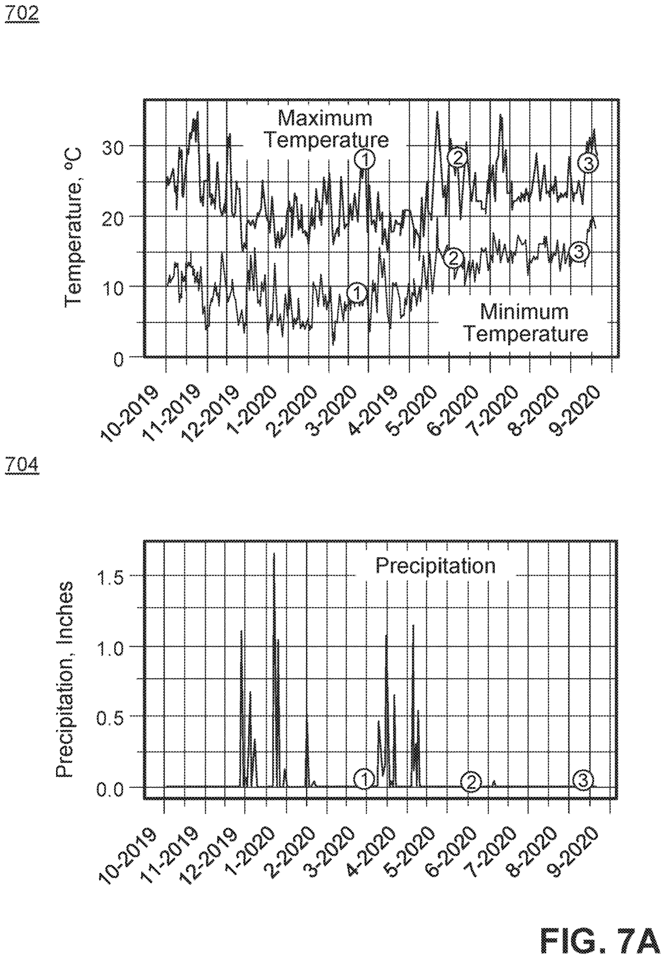

Avocado fruits can be collected from trees on at a farm or other growing area. As shown in the aerial view 602 in FIG. 6A, samples were collected from 13 trees located in Block 7 of the farm. The fruit can be collected periodically throughout a growing season. All fruit can be collected by gently clipping the fruit with stainless steel clippers that were frequently sterilized with a spray of 70% EtOH throughout the harvest, leaving ~2-4cm of pedicel. In the example process 600, at each collection timepoint, 30 fruit can be harvested from each tree and transported to a research center in crates (having approximately a 15 minute transport time). A total of 1,050 fruit can be collected from these trees. Climate data for harvest dates can also be retrieved from a data store, as shown in graphs 702 and 704 in FIG. 7A. The timepoints analyzed in the example process 600 can be from ~330 DAF, ~430 DAF, and ~520 DAF.

Upon arrival at the research center, the fruit can be arranged onto produce flats and allowed to equilibrate to ambient temperature overnight. Approximately 24 hours after harvest, fruit mass (g) and respiration rate (mL CO₂/kg-hr) can be individually measured for all samples (n = 30 per tree per timepoint). Fruit respiration can be measured by sealing the fruit in custom air-tight pods equipped with CO₂ sensors for 4 minutes, measuring CO₂ concentration every 5 seconds. Percent dry matter of each fruit can be determined using a Near-Infrared hand-held spectrometer with a custom dry matter prediction algorithm. Images of an exterior quality of the fruit can also be taken. At this point, a subset of fruit (n = 4 per tree per timepoint) can also be destructively sampled for RNA extraction while the remainder of the fruit (n = 24) can be allowed to ripen in ambient conditions. The length of time (in days) required for fruit to reach 50 shore (as measured with a hand-held durometer) can be deemed the time to ripe. Upon reaching 50 shore, fruit can be cut in half, inspected for quality defects (including SER) and imaged, as shown in the image data 606.

At the end of all collections, the incidence of SER in ripened fruit in individual trees at each timepoint can be determined. Trees that produced fruit with a marked increase in the incidence of SER across the season can be designated as "high incidence" while trees that produced fruit with low incidences of SER can be chosen as control or "low incidence" for comparison. Three "high incidence" (A1, C1 and D1) and three "low incidence" (A4, C2, and D2) trees can be chosen for in-depth transcriptomics analysis. Refer to the heatmap 604 in FIG. 6B.

As mentioned previously, at each timepoint, 4 fruit from each tree can be sampled destructively 24 hours after harvest by cutting a cross section of the fruit (which can include seed, endocarp, mesocarp, and exocarp) and grinding to a fine powder under liquid nitrogen. These cross sections can include skin, flesh, and seed. The samples can be stored at -80° C until used for RNA extractions.

RNA can then be extracted from each individual sample from the "high incidence" and "low incidence" trees collected at each of the timepoints using a cetyl trimethyl ammonium bromide (CTAB) buffer-based extraction protocol with bead clean up. RNAseq libraries can then be prepared and pooled by timepoint for sequencing. Libraries can be run, in some implementations, at 75 cycles (high output). File quality can be assessed and aligned to a reference genome for the particular avocados. Bam file generation and indexing can also be performed.

Sequence reads can also be assigned to genomic features. Read count tables can be prepared separately for each timepoint. Differential expression can be determined between the high and low incidence trees at each timepoint. Differentially expressed genes can be filtered for low counts (only genes with >10 counts were kept) and for a p-value of <0.05.

Principal component analysis can also be performed and visualized on the transformed, normalized DeSeq matrix. In preparation for heatmap visualization, the matrix of fold change values for the top, up, and down regulated genes at each timepoint (filtered for p-value < 0.05) can be normalized to each genes' values across the samples. An R function *hclust* can be used to cluster the genes by expression pattern within each timepoint (using method = complete). An R function *pheatmap* can be used in some implementations to visualize how individual samples cluster based on gene expression patterns.

The clustering of differentially expressed genes by expression trends over time can be performed and visualized. Such functions may calculate gene similarity across samples within conditions and over time. The relative gene expression can be normalized to a Z-score where values can be centered to a mean and scaled to standard deviation of each gene. The genes analyzed for trends over time can be limited, in some implementations, to top 500 upregulated differentially expressed genes and top 500 downregulated differentially expressed genes with p-values < 0.05. Gene clusters examined can be limited to those with gene counts > 20.

Gene ontology analysis can be performed on lists of top differentially regulated avocado genes by timepoint or by expression cluster using the pre-determined homologous genes in *Arabidopsis* for each avocado transcript. These lists can be used as input to an enrichment analysis tool that can map a provided list of genes to known functional information sources, detecting statistically significant enriched biological processes, pathways, regulatory motifs, and protein complexes. Adjusted enrichment p-values for each enrichment classification can be determined and outputted (e.g., reported).

Overall, FIGs. 6A-C demonstrates the location (e.g., area) of sampled trees and percent incidence of SER in the fruit collected from each tree at each timepoint. At the first collection date, 330 DAF, no SER was observed. Over the next two collections, SER incidence increased on average and increased most significantly in fruit from the trees A1, C1 and D1. The samples from these trees can accordingly be assigned to a "high incidence" group. Nearby trees having minimal to no SER across collections, A4, C2 and D2, can be assigned to a "low incidence" group as a control comparison.

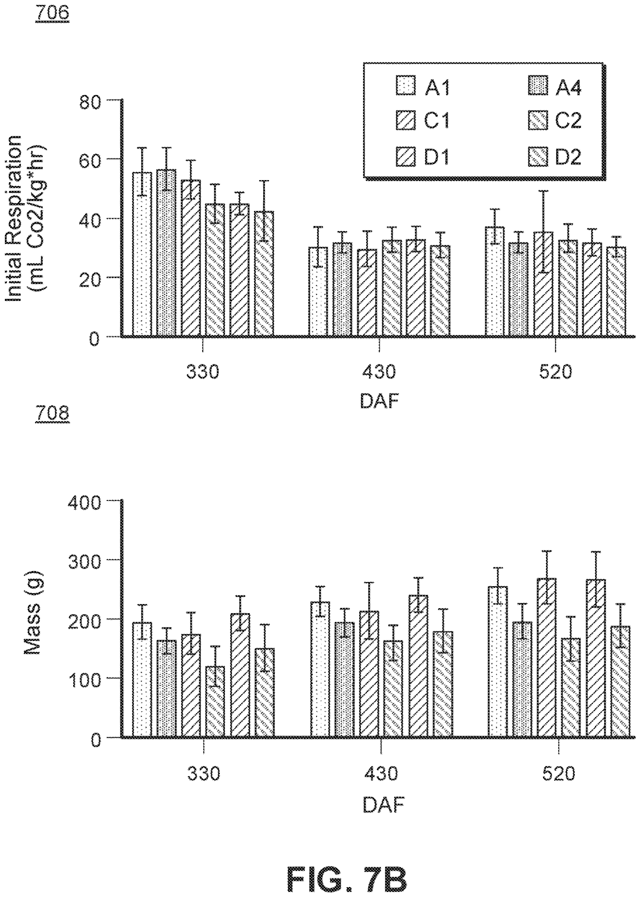

FIGs. 7A-B depict graphical depictions of example physical attributes of the plants of FIG. 6 that can be collected at different times. Graph 702 in FIG. 7A is a plot of maximum and minimum temperature throughout collection of fruit described herein, with sampling timepoints labeled as 1, 2, and 3. Graph 704 in FIG. 7A is a plot of precipitation measured for the area throughout collection of the fruit described herein, with the sampling timepoints labeled 1, 2, and 3. Graph 706 in FIG. 7B depicts average initial respiration per fruit collected at 330 DAF, 430 DAF, and 520 DAF. Graph 708 in FIG. 7B depicts average mass of the fruit collected from the chosen high and low SER incidence trees at each timepoint with standard deviation plotted as error bars.

Referring to both FIGs. 7A-B, respiration rate 24 hours after harvest can be highest for fruit from all trees at the first timepoint in February, 330 DAF (e.g., refer to graph 706 in FIG. 7B). This could be attributed to temperatures on average being lower in the beginning of the year (e.g., refer to graph 702 in FIG. 7A) and/or to a developmental stage of the fruit at the time, as rapid growth and/or cell division may necessitate increased metabolism and therefore respiration. There may be no significant differences observed in average dry matter or respiration rate between trees with high and low incidence of SER, however there can be a trend observed between average mass of individual fruit and percent incidence of SER. As days after flowering increase, fruit mass can also increase (e.g., refer to graph 708 in FIG. 7B), however, the average fruit mass may be consistently higher for fruit from the high incidence SER group. This trend can be most significant at the third collection (520 DAF), where the average fruit mass from A1, C1 and D1 are greater than 250 g while the average fruit mass for A4, C2 and D2 are all less than 200 g (e.g., refer to graph 708 in FIG. 7B).

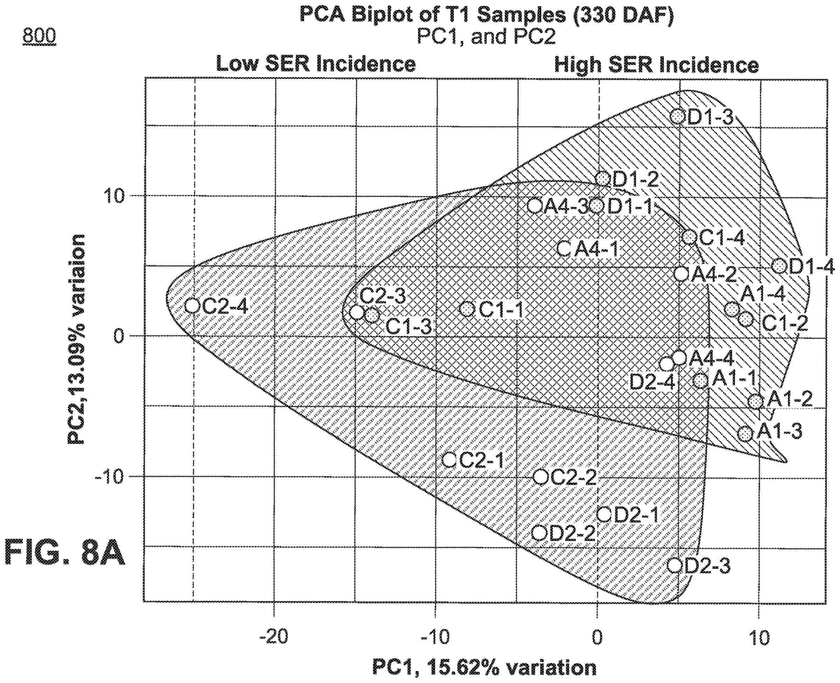

FIGs. 8A-C depict biplots of principal component analysis (PCA) of normalized gene expressions across all times of data collection for the plants of FIGs. 6A-C. In particular, biplot 800 depicts the first 2 principal components from PCA of the gene expression data at 330 DAF for both low and high SER incidence groups. Biplot 802 depicts the first 2 principal components from PCA of the gene expression data at 430 DAF for both low and high SER incidence groups. Biplot 804 depicts the first 2 principal components from PCA of the gene expression data at 520 DAF for both low and high SER incidence groups.

Referring to the biplots 800-804 in FIGs. 8A-C, across all three sequencing runs, 5-6 million reads can be obtained per sample, with percent reads identified (%PF) and percent QScore >30 (%>=Q30) over 90 and 95% respectively. Upon alignment to the *Persea americana* cv. Hass genome, an average of 81.4, 80.8 and 80.9% of reads can be successfully assigned to annotated regions of the genome for the t1 (330 DAF), t2 (430 DAF) and t3 (520 DAF) data sets respectively.

Visualization of the PCA of normalized expression across all timepoints using a pairsplot can illustrate that the strongest separation by PCA occurs between timepoints, particularly in PC1 - PC2. There can be some slight separation of the low and high SER groups in PC3 and PC4. However, when PCA is performed within each individual timepoint and visualized using biplots, slightly more defined separation between the low and high SER samples can be observed across PC1 and PC2, as shown in FIGs. 8A-C. This separation can appear most distinct at the latest timepoint of 520 DAF.

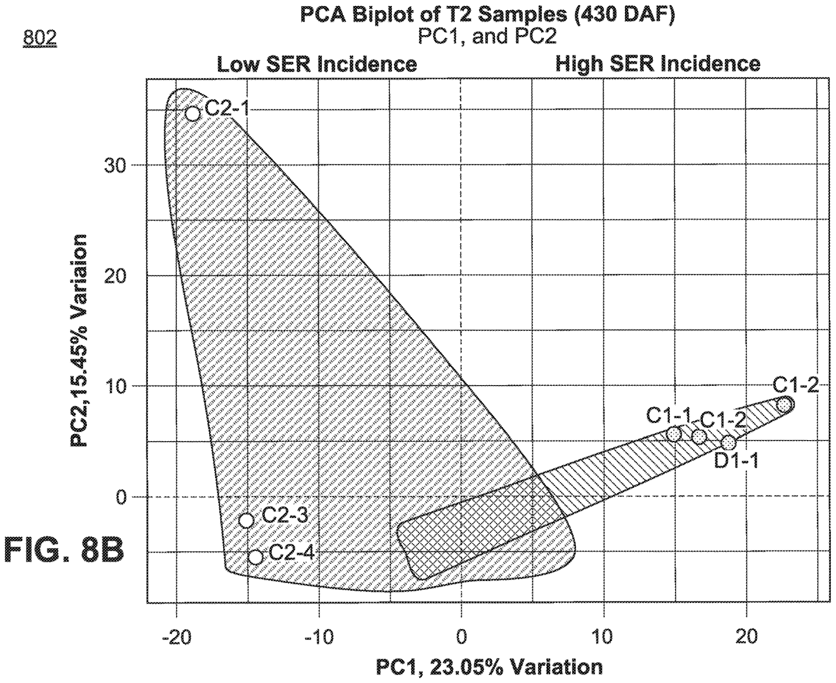

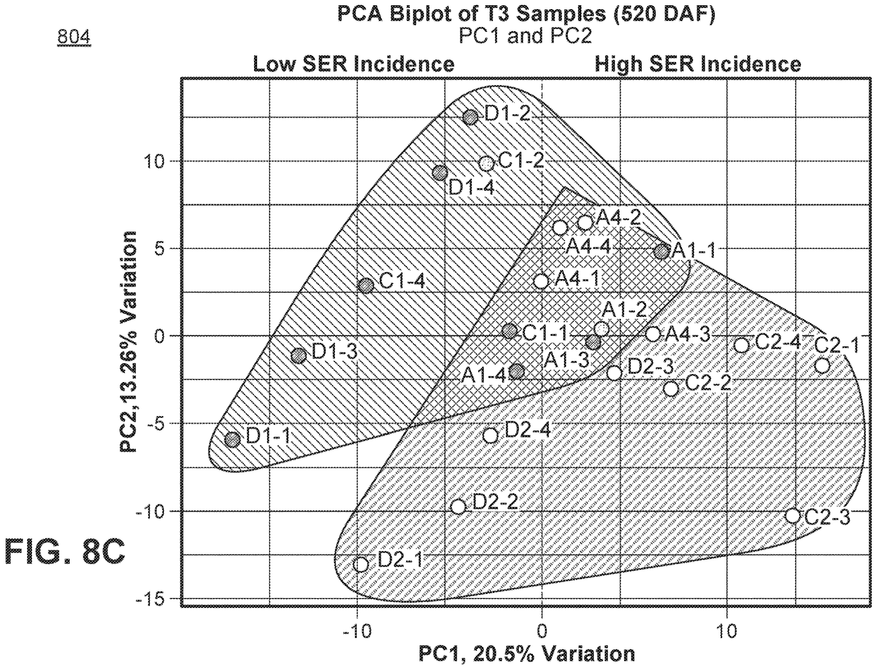

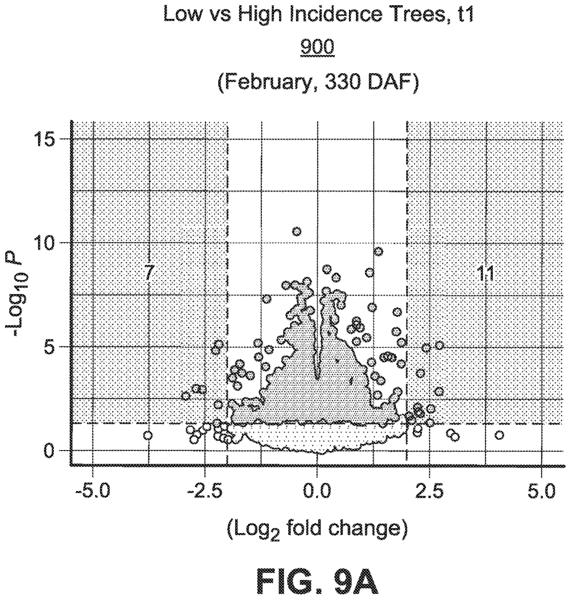

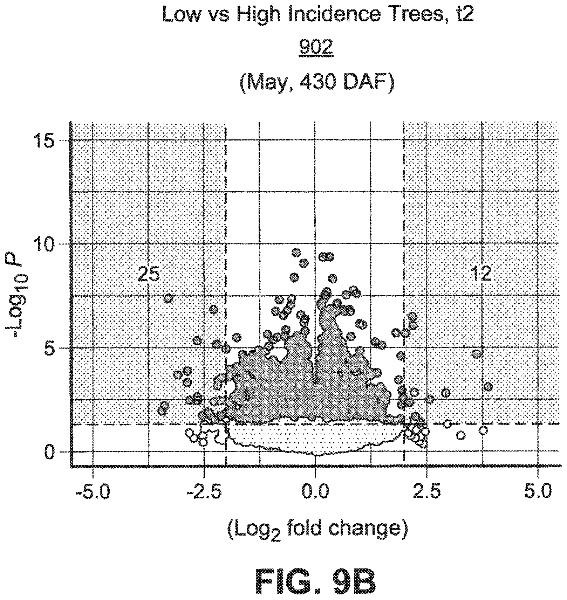

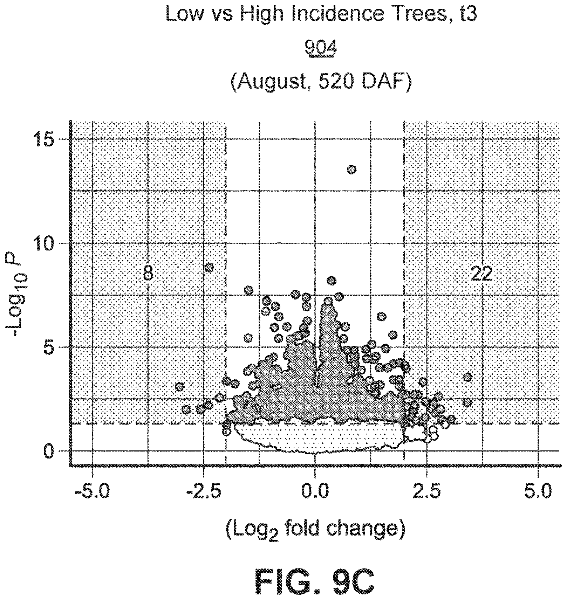

FIGs. 9A-C depict volcano plots of normalized gene expressions for the plants of FIGs. 6A-C. Volcano plot 900 in FIG. 9A shows differential gene expression between low and high incidence tree groups at 330 DAF, volcano plot 902 in FIG. 9B shows differential gene expression between low and high incidence tree groups at 430 DAF, and volcano plot 904 in FIG. 9C shows differential gene expression between low and high incidence tree groups at 520 DAF. The plots 900-904 therefore depict normalized gene expression (Log₂ fold change) versus statistical significance (-Log₁₀P). In each of the plots 900-904, differentially expressed genes with a Log₂ fold change > |2| and a p value < 0.05 fall into the patterned quadrants. Numbers in these quadrants represent the number of genes that meet these qualifications. Thus, in FIG. 9A, 7 genes meet the qualifications for the first quadrant and 11 genes meet the qualifications for the second quadrant, in FIG. 9B, 25 genes and 12 genes, respectively, and in FIG 9C, 8 genes and 22 genes, respectively.

Referring to FIGs. 9A-C, at all three timepoints, comparing the high incidence tree group (A1, C1, and D1) to the low incidence tree group (A4, C2, and D2) can show differences in gene expression. At t1, 863 genes can be upregulated (4% of genes identified) and 963 can be downregulated (4.4% of genes identified) in the high incidence trees. At t2, 1012 genes can be upregulated (4.8% of genes identified) and 786 can be downregulated (3.7 % of genes identified) in the high incidence trees. At t3, 234 genes can be upregulated (1% of genes identified) and 166 can be downregulated (0.08% of genes identified) in the high incidence trees. When these DEGs are filtered for a p-value of <0.05 and a fold change of < -2 or > 2, the up and down regulated genes in high incidence trees in t1, t2 and t3 can be 11 up and 7 down, 12 up and 25 down, and 22 up and 8 down, respectively.

In some implementations, heatmap visualizations (not depicted) of top 30 up- and down- regulated genes at each timepoint (pre-filtered for a p-value of < 0.05 prior to sorting by fold change) may show that the individual fruit expression patterns from trees that were labeled as high incidence cluster together. This may suggest that these trees can be assigned as high or low incidence based on gene expression patterns for these genes in the fruit as early as the first timepoint in February, 330 DAF and 6 months prior to late season harvest. Notably, there may be little overlap in these genes across the three timepoints. Only 4 genes can be conserved across these three groupings: maker-Ctg1243-augustus-gene-0.16-mRNA-1, maker-Ctg0870-augustus-gene-0.10-mRNA-1, augustus_masked-Ctg0285-processed-gene-0.0-mRNA-1 and maker-Ctg0255-augustus-gene-4.16-mRNA-1. The predicted nearest tomato (*S*. *lycopersicum)* and Arabidopsis *(A. thaliana)* homologues for each of these genes can be identified by blast analysis. These results may suggest that the responses to latent fungal pathogenesis at the transcriptome level change throughout the phases of fruit development on the tree.

Statistical enrichment analysis of the top 30 up and down regulated genes at each timepoint can use a list of *Arabidopsis* homologs as input. The results can be filtered for gene ontology terms with a p-value of less than or equal to 0.05 and a false discovery rate (FDR) of less than 1. There can be, in the example of FIGs. 6A-C, 20, 35, and 79 gene ontology terms that meet these criteria for the t1, t2 and t3 timepoints, respectively. This analysis can provide high level insight into most significantly represented biological processes, molecular functions, and cellular component classifications in this transcript subset that may exhibit strong differentiation between the low and high SER trees.

As expected from limited overlap in specific genes between timepoints in the top 30 up- and down- regulated gene sets, there may be limited overlap between the most significant gene ontology classifications, with t1 and t2 containing more overlap than either timepoint with t3. The t1 and t2 significant GO datasets, for example, both may contain the biological process terms response to other organism (GO:0051707) and response to external biotic stimulus (GO:0043207) along with the cellular component term cell periphery (GO:0071944) and molecular function term transporter activity (GO:0005215), while none of these terms may overlap with t3. In addition to these terms, t2 can have additional terms related to oxidoreductase activity (GO:0016491), response to oxidative stress (GO:0006979), plant organ development (GO:0099402), response to osmotic stress (GO:0006970), defense response to other organism (GO:0098542), and aromatic compound biosynthetic process (GO:0019438) that may not be present in the t1 dataset. The t3 significant GO list may also contain the terms oxidoreductase activity (GO:0016491) and aromatic compound biosynthetic process (GO:0019438), as well as terms related to plant organ development (GO:0099402), such as post-embryonic development (GO:0009791) and reproductive structure development (GO:0048608) that can overlap with t2. Largely, t3 may not overlap with t1 or t2. Instead, the t3 GO list can contain terms related to hormonal signaling, including response to hormone (GO:0009725), cellular response to hormone stimulus (GO:0032870), and hormone-mediated signaling pathway (GO:0009755). Additionally, the t3 dataset can contain many GO terms related to transcriptional regulation that do not appear in the t1 or t2 datasets. These can include but are not limited to transcription (GO:0006351), regulation of transcription (GO:0006355), DNA binding (GO:0003677), RNA biosynthetic process (GO:0032774), and regulation of RNA biosynthetic process (GO:2001141).

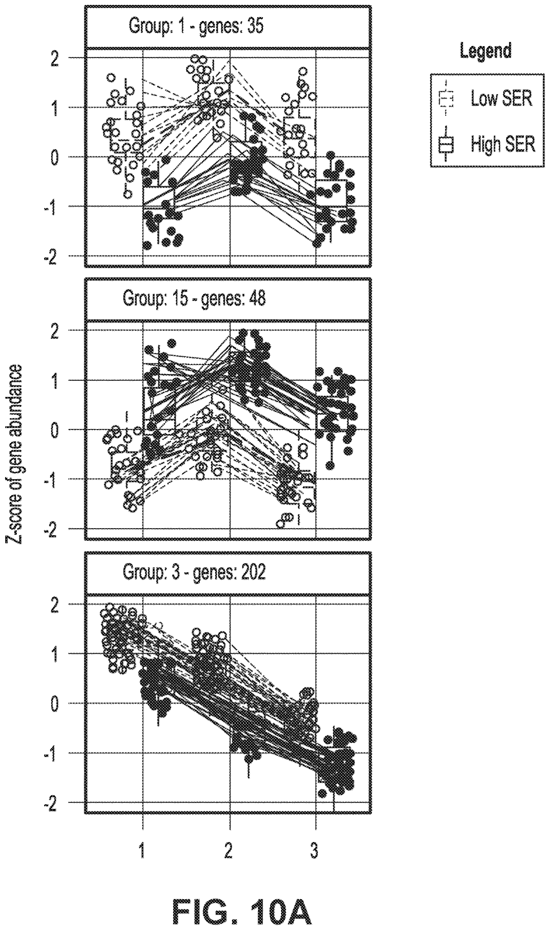

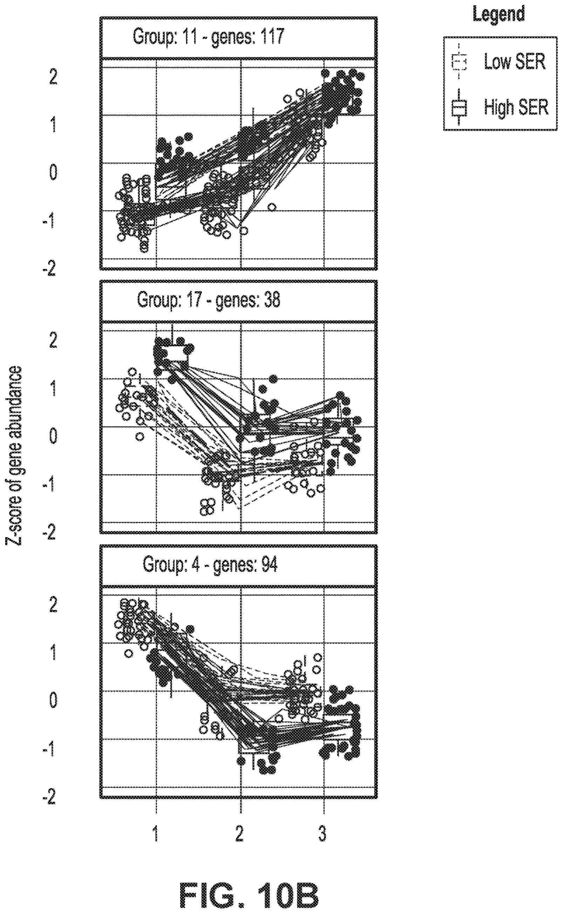

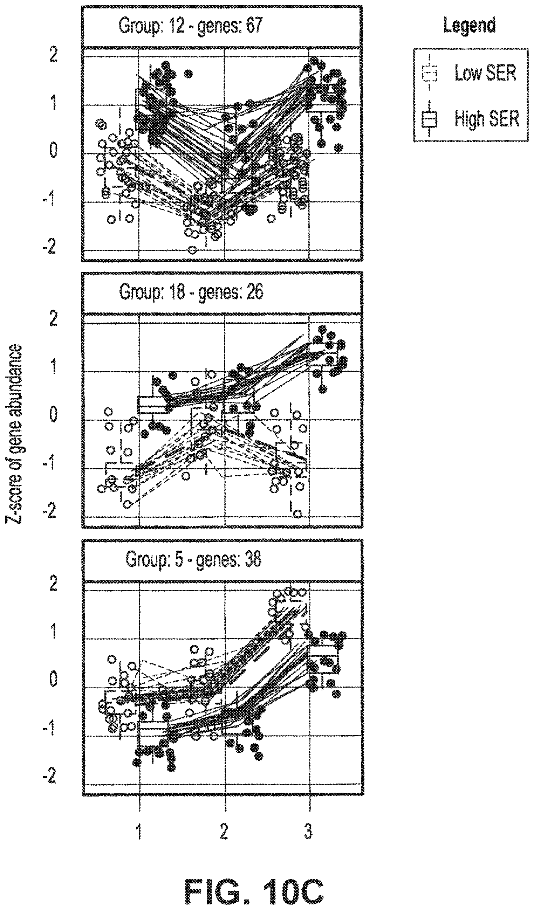

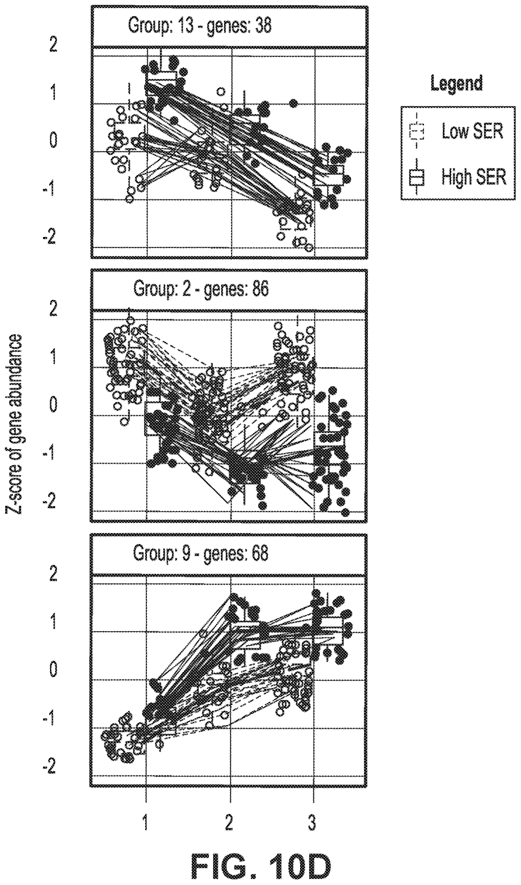

FIGs. 10A-D depicts normalized gene expression over time for selected gene clusters with strong time and condition trends for the plants of FIGs. 6A-C. Referring to all the FIGs. 10A-D, numbers 1, 2, and 3 on the x axis indicate each sampling timepoint at 330 DAF, 430 DAF, and 520 DAF. The clustering of differentially expressed genes by expression trends over time can be performed and visualized using known computing techniques. Such techniques can calculate gene similarity across samples within conditions and over time. Relative expression can be normalized to a Z-score where values can be centered to the mean and scaled to the standard deviation of each gene. The genes analyzed for trends over time can be limited to the top 500 upregulated and top 500 down-regulated differentially expressed genes with p-values <0.05. Gene clusters visualized can be limited to those with gene counts >20. 16 gene clusters met these criteria and are shown in FIGs. 10A-D. g:GOSt can be used to perform statistical enrichment analysis using a list of *Arabidopsis* homologs to the *P. americana* genes categorized into each cluster. A summary of each group's top GO categories is shown in Table 2.

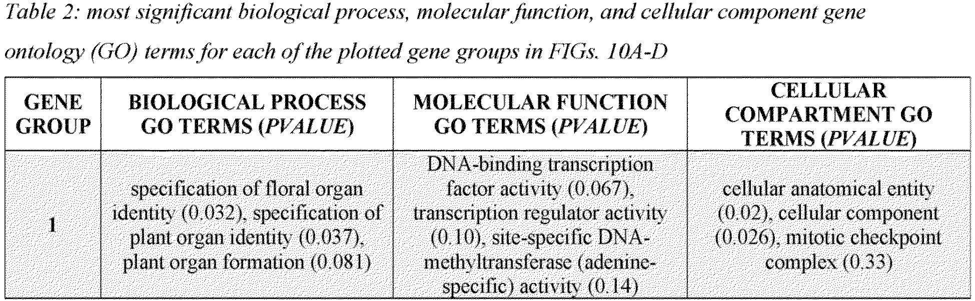

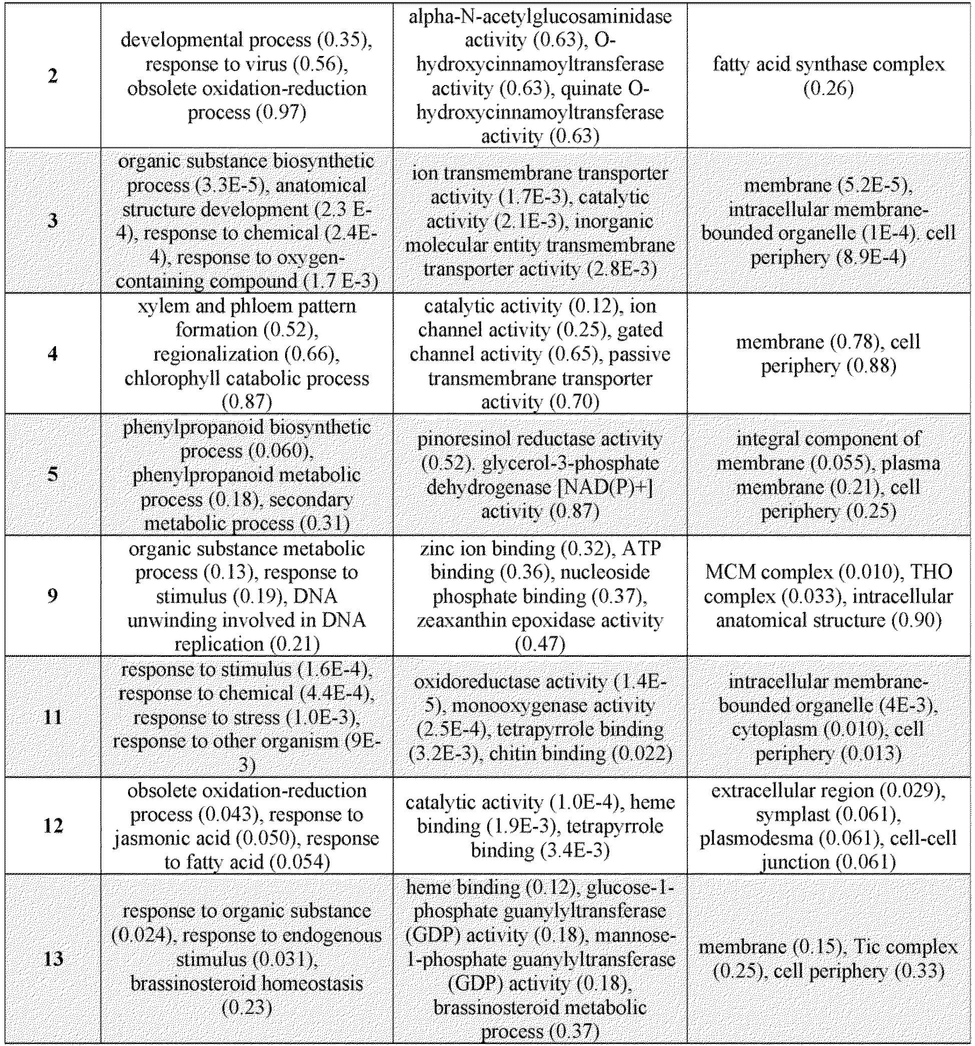

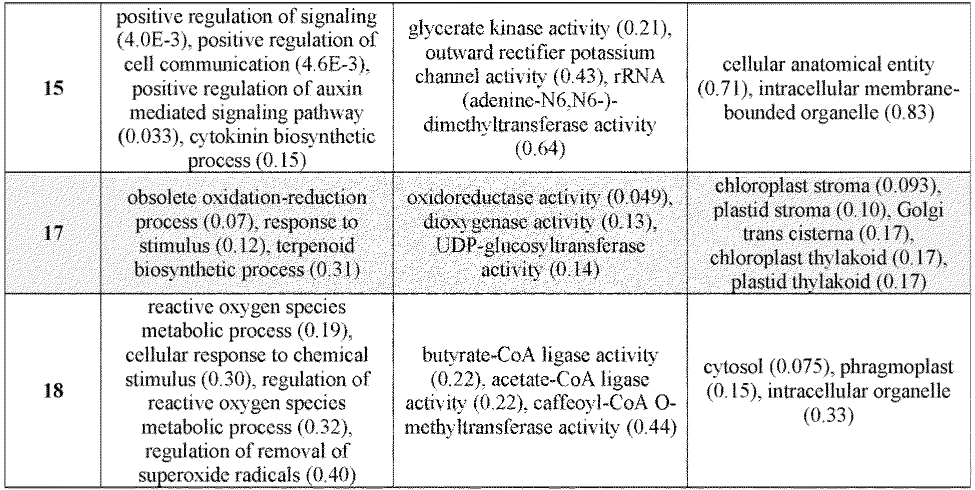

Notably, as shown in FIGs. 10A-D and Table 2, there can be several groups that exhibit an increasing trend over time and have higher relative gene amounts in fruit from high infection rate trees (Group 9, Group 11, Group 12 and Group 18). The genes and GO annotations for these groups can be examined for pathways characteristic of the plant-pathogen response and Group 11 can be found to have a notable number of terms related to these pathways. For example, some the top biological process GO categories with p-values much smaller than 0.05 in Group 11 can be response to stimulus (GO:0050896), response to chemical (GO:0042221), and response to other organism (GO:0051707), with examples of genes from these categories including but not limited to predicted endochitinase 4 (maker-Ctg0022-augustus-gene-3.7), pathogenesis-related protein PR1 precursor (augustus_masked-Ctg0281-processed-gene-0.6), and osmotin-like pathogenesis-related protein R (augustus_masked-Ctg2009-processed-gene-0.2) plotted in FIGs. 10A-D. Group 18 additionally can contain GO groups involved in the response to stress such as reactive oxygen species metabolic process (GO:0072593) and regulation of removal of superoxide radicals (GO:2000121). Furthermore, group 12 can have an over representation of genes involved in the response to jasmonic acid (GO:0009753), which can be established to be involved in plant defense signaling. Thus, key differences in gene expression over time may exist between the high and low SER tree groups, which can be attributed to shifts in characteristic pathogen response pathways.

In many of the gene groups where the high SER samples exhibited reduced expression compared to the low SER group (Group 1, Group 2 and Group 4), there can be a general over representation of genes involved in cell development and differentiation. For example, the most significant biological process GO terms in Group 1 can be specification of floral organ identity (GO:0010093) and specification of plant organ identity (GO:0090701) while the top two in Group 4 can be xylem and phloem pattern formation (GO:0010051) and regionalization (GO: 0003002). This can suggest that fruit from trees with more SER can have differences in developmental patterns. Additionally, Group 5 contains a high representation of genes involved in the phenylpropanoid biosynthetic process (GO:0009699). Phenylpropanoid compounds can be known to be involved in plant defense. Thus, higher expression of enzymes involved in phenylpropanoid biosynthesis can make the low SER fruit less susceptible to SER infection. Moreover, the phenylpropanoid pathway can create the precursors for lignin biosynthesis and, therefore, may be related to the differences in developmental gene expression seen in Groups 1, 2, and 4.

FIGs. 6-10 demonstrate that differential gene expression analysis can reveal significantly different up- and down- regulated genes in fruit samples from trees grouped by high and low SER incidence. These trees can be classified into high or low SER incidence groups based on manifestation of SER symptoms in ripened fruit of harvest maturity. It can be observed that when fruit of lower maturities (less than -30% dry matter) are harvested at the first two timepoints and allowed to ripen, little to no SER may manifest, while the more mature fruit at the latest timepoint can develop significant levels of SER. This trend can be attributed to increases in average temperature leading up to the final timepoint. The gene expression data may demonstrate that, even at these early timepoints prior to manifestation of symptoms, there are measurable differences in the transcriptomic landscape of fruit from a high incidence tree, which can facilitate pre-harvest prediction of latent infection.

Moreover, as described herein with regards to FIGs. 6-10, the specific genes and processes most differentially altered in infected trees can shift over the time course of fruit development. This can be illustrated through minimal overlap between the top 30 up- and top 30 down- regulated genes capable of clustering fruit expression trends into the expected high and low SER groups. The analysis of the groups of genes can be differentially regulated over time, which may further demonstrate dynamic shifts in relative abundance of gene transcripts across the three timepoints sampled. Even so, gene ontology analysis of these grouped expression trends over time can enable isolation of groups of known pathogen response genes, including osmotin-like proteins and chitinases, which can show consistent and/or increasing trends in differential expression in the high SER tree groups over time. Moreover, genes involved in jasmonic acid signaling and reactive oxygen species metabolism can be identified to be upregulated in fruit from high incidence trees at early timepoints (e.g., refer to Group 12 and Group 18 in FIGs. 10A-D, Table 2). Reactive oxygen species and jasmonic acid signaling events can be known mechanisms deployed in the early host defense response against fungal pathogens. This can suggest that the fruit from trees with higher SER incidence can, in fact, exhibit a heightened response to the presence of the SER fungal pathogens long before manifestation of infection symptoms.

An average incidence of SER post-harvest, even in the high incidence trees, can be maximally observed to be 17% in tree C1. With 4 individual fruit samples per tree, the likelihood that these particular fruit would be the individuals that develop SER symptoms is low. Therefore, the observed differences in gene expression between fruit from low and high incidence trees in the chosen samples can be interpreted as a systemic response throughout that tree to the presence of SER fungi.

Additionally, analysis of genes grouped by differential expression over time (e.g., refer to FIGs. 10A-D, Table 2) additionally may suggest differences in fruit development between the fruit from high and low SER incidence trees. Specifically, the high SER fruit may exhibit reduced expression of gene groups involved in cell development and differentiation. The measured physical difference between the high and low SER groups can be a slight difference in average mass of harvested fruit over the three timepoints (e.g., refer to FIG. 7B), with the high SER trees producing fruit of higher average mass.

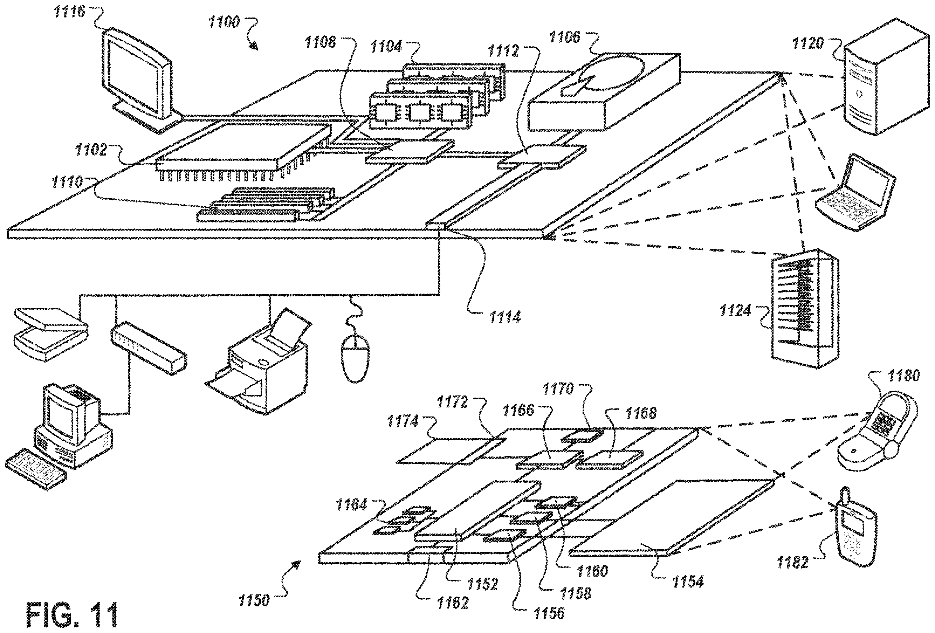

FIG. 11 is a block diagram of system components that can be used to implement a system for detecting latent infection. Computing device 1100 is intended to represent various forms of digital computers, such as laptops, desktops, workstations, personal digital assistants, servers, blade servers, mainframes, and other appropriate computers. Computing device 1150 is intended to represent various forms of mobile devices, such as personal digital assistants, cellular telephones, smartphones, and other similar computing devices. Additionally, computing device 1100 or 1150 can include Universal Serial Bus (USB) flash drives. The USB flash drives can store operating systems and other applications. The USB flash drives can include input/output components, such as a wireless transmitter or USB connector that can be inserted into a USB port of another computing device. The components shown here, their connections and relationships, and their functions, are meant to be examples only, and are not meant to limit implementations of the invention described in this document.

Computing device 1100 includes a processor 1102, memory 1104, a storage device 1108, a high-speed interface 1108 connecting to memory 1104 and high-speed expansion ports 1110, and a low speed interface 1112 connecting to low speed bus 1114 and storage device 1108. Each of the components 1102, 1104, 1108, 1108, 1110, and 1112, are interconnected using various busses, and can be mounted on a common motherboard or in other manners as appropriate. The processor 1102 can process instructions for execution within the computing device 1100, including instructions stored in the memory 1104 or on the storage device 1108 to display graphical information for a GUI on an external input/output device, such as display 1116 coupled to high speed interface 1108. In other implementations, multiple processors and/or multiple buses can be used, as appropriate, along with multiple memories and types of memory.

Also, multiple computing devices 1100 can be connected, with each device providing portions of the necessary operations, e.g., as a server bank, a group of blade servers, or a multiprocessor system.

The memory 1104 stores information within the computing device 1100. In one implementation, the memory 1104 is a volatile memory unit or units. In another implementation, the memory 1104 is a non-volatile memory unit or units. The memory 1104 can also be another form of computer-readable medium, such as a magnetic or optical disk.

The storage device 1108 is capable of providing mass storage for the computing device

1100. In one implementation, the storage device 1108 can be or contain a computer-readable medium, such as a floppy disk device, a hard disk device, an optical disk device, or a tape device, a flash memory or other similar solid-state memory device, or an array of devices, including devices in a storage area network or other configurations. A computer program product can be tangibly embodied in an information carrier. The computer program product can also contain instructions that, when executed, perform one or more methods, such as those described above. The information carrier is a computer- or machine-readable medium, such as the memory 1104, the storage device 1108, or memory on processor 1102.

The high-speed controller 1108 manages bandwidth-intensive operations for the computing device 1100, while the low speed controller 1112 manages lower bandwidth intensive operations. Such allocation of functions is only an example. In one implementation, the high-speed controller 1108 is coupled to memory 1104, display 1116, e.g., through a graphics processor or accelerator, and to high-speed expansion ports 1110, which can accept various expansion cards (not shown). In the implementation, low-speed controller 1112 is coupled to storage device 1108 and low-speed expansion port 1114. The low-speed expansion port, which can include various communication ports, e.g., USB, Bluetooth, Ethernet, wireless Ethernet can be coupled to one or more input/output devices, such as a keyboard, a pointing device, microphone/speaker pair, a scanner, or a networking device such as a switch or router, e.g., through a network adapter. The computing device 1100 can be implemented in a number of different forms, as shown in the figure. For example, it can be implemented as a standard server 1120, or multiple times in a group of such servers. It can also be implemented as part of a rack server system 1124. In addition, it can be implemented in a personal computer such as a laptop computer 1122.

Alternatively, components from computing device 1100 can be combined with other components in a mobile device (not shown), such as device 1150. Each of such devices can contain one or more of computing device 1100, 1150, and an entire system can be made up of multiple computing devices 1100, 1150 communicating with each other.

The computing device 1100 can be implemented in a number of different forms, as shown in the figure. For example, it can be implemented as a standard server 1120, or multiple times in a group of such servers. It can also be implemented as part of a rack server system 1124. In addition, it can be implemented in a personal computer such as a laptop computer 1122.

Alternatively, components from computing device 1100 can be combined with other components in a mobile device (not shown), such as device 1150. Each of such devices can contain one or more of computing device 1100, 1150, and an entire system can be made up of multiple computing devices 1100, 1150 communicating with each other.

Computing device 1150 includes a processor 1152, memory 1164, and an input/output device such as a display 1154, a communication interface 1166, and a transceiver 1168, among other components. The device 1150 can also be provided with a storage device, such as a micro-drive or other device, to provide additional storage. Each of the components 1150, 1152, 1164, 1154, 1166, and 1168, are interconnected using various buses, and several of the components can be mounted on a common motherboard or in other manners as appropriate.

The processor 1152 can execute instructions within the computing device 1150, including instructions stored in the memory 1164. The processor can be implemented as a chipset of chips that include separate and multiple analog and digital processors. Additionally, the processor can be implemented using any of a number of architectures. For example, the processor 1110 can be a CISC (Complex Instruction Set Computers) processor, a RISC (Reduced Instruction Set Computer) processor, or a MISC (Minimal Instruction Set Computer) processor. The processor can provide, for example, for coordination of the other components of the device 1150, such as control of user interfaces, applications run by device 1150, and wireless communication by device 1150.

Processor 1152 can communicate with a user through control interface 1158 and display interface 1156 coupled to a display 1154. The display 1154 can be, for example, a TFT (Thin-Film- Transistor Liquid Crystal Display) display or an OLED (Organic Light Emitting Diode) display, or other appropriate display technology. The display interface 1156 can comprise appropriate circuitry for driving the display 1154 to present graphical and other information to a user. The control interface 1158 can receive commands from a user and convert them for submission to the processor 1152. In addition, an external interface 1162 can be provided in communication with processor 1152, so as to enable near area communication of device 1150 with other devices.

External interface 1162 can provide, for example, for wired communication in some implementations, or for wireless communication in other implementations, and multiple interfaces can also be used.

The memory 1164 stores information within the computing device 1150. The memory 1164 can be implemented as one or more of a computer-readable medium or media, a volatile memory unit or units, or a non-volatile memory unit or units. Expansion memory 1174 can also be provided and connected to device 1150 through expansion interface 1172, which can include, for example, a SIMM (Single In Line Memory Module) card interface. Such expansion memory 1174 can provide extra storage space for device 1150, or can also store applications or other information for device 1150. Specifically, expansion memory 1174 can include instructions to carry out or supplement the processes described above, and can also include secure information. Thus, for example, expansion memory 1174 can be provided as a security module for device 1150, and can be programmed with instructions that permit secure use of device 1150. In addition, secure applications can be provided via the SIMM cards, along with additional information, such as placing identifying information on the SIMM card in a non-hackable manner.

The memory can include, for example, flash memory and/or Non-volatile random-access memory (NVRAM) memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 1164, expansion memory 1174, or memory on processor 1152 that can be received, for example, over transceiver 1168 or external interface 1162.

Device 1150 can communicate wirelessly through communication interface 1166, which can include digital signal processing circuitry where necessary. Communication interface 1166 can provide for communications under various modes or protocols, such as GSM voice calls, SMS, EMS, or MMS messaging, CDMA, TDMA, PDC, WCDMA, CDMA2000, or GPRS, among others. Such communication can occur, for example, through radio-frequency transceiver 1168. In addition, short-range communication can occur, such as using a Bluetooth, Wi-Fi, or other such transceiver (not shown). In addition, GPS (Global Positioning System) receiver module 1170 can provide additional navigation- and location-related wireless data to device 1150, which can be used as appropriate by applications running on device 1150.

Device 1150 can also communicate audibly using audio codec 1160, which can receive spoken information from a user and convert it to usable digital information. Audio codec 1160 can likewise generate audible sound for a user, such as through a speaker, e.g., in a handset of device 1150. Such sound can include sound from voice telephone calls, can include recorded sound, e.g., voice messages, music files, etc. and can also include sound generated by applications operating on device 1150.

The computing device 1150 can be implemented in a number of different forms, as shown in the figure. For example, it can be implemented as a cellular telephone 1180. It can also be implemented as part of a smartphone 1182, personal digital assistant, or other similar mobile device.

Various implementations of the systems and methods described here can be realized in digital electronic circuitry, integrated circuitry, specially designed ASICs (application specific integrated circuits), computer hardware, firmware, software, and/or combinations of such implementations. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device, e.g., magnetic discs, optical disks, memory, Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device, e.g., a CRT (cathode ray tube) or LCD (liquid crystal display) monitor for displaying information to the user and a keyboard and a pointing device, e.g., a mouse or a trackball by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The systems and techniques described here can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the systems and techniques described here, or any combination of such back end, middleware, or front end components. The components of the system can be interconnected by any form or medium of digital data communication, e.g., a communication network. Examples of communication networks include a local area network ("LAN"), a wide area network ("WAN"), and the Internet.

The computing system can include clients and servers. A client and server are generally remote from each other and typically interact through a communication network. The relationship of client and server arises by virtue of computer programs running on the respective computers and having a client-server relationship to each other.

## Claims

1. A method for identifying pre-harvest latent infection in a plant, the method comprising:
obtaining, by a processor, data describing a level of expression of one or more infection biomarkers present in the plant, the data including a list of one or more variants, the infection biomarkers indicating a likelihood of infection in the plant, wherein the one or more variants describe differences between a read sequence of the plant and a reference genome of a healthy plant of a same type as the plant, wherein the read sequence of the plant represents an order of nucleotides in a nucleic acid of the plant;
selecting, by the processor, one or more machine learning models based on the obtained data, wherein the one or more machine learning models were previously trained, using data that correlates other data and determined one or more infection biomarkers of one or more other plants, to generate output indicating a likelihood that the plant is developing a latent infection pre-harvest, wherein the one or more machine learning models were trained using a process comprising:
inputting, from a training data set and into the one or more machine learning models, (i) non-invasive measurements of the one or more other plants, (ii) invasive measurements of the one or more other plants, (iii) known plant information for the one or more other plants, and (iv) affirmative infection identifications for the one or more other plants, and
determining predicted likelihoods that the one or more other plants are developing the latent infection pre-harvest based on inputting (i)-(iv) into the one or more machine learning models, and
outputting the one or more machine learning models for runtime use;
generating, by the processor, output indicating the likelihood that the plant is developing the latent infection pre-harvest based on applying the one or more machine learning models to the data;
determining, by the processor, that the plant has a latent infection based on the output exceeding a predetermined threshold range;
determining, by the processor, one or more operations to mitigate the latent infection in the plant; and
outputting, by the processor, an indication that the plant has the latent infection and the one or more determined operations.

2. The method of claim 1, wherein determining, by the processor, the one or more operations to mitigate the latent infection in the plant comprises determining, for the plant and one or more other similarly sourced plants, at least one of (i) a modified harvest schedule that is earlier in a growing season, (ii) instructions to apply a predetermined amount of pesticide pre-harvest, and (iii) when the plant and the one or more other similarly sourced plants are nearing an end of respective healthy production lifespans, wherein the one or more machine learning models were previously trained, using data from the training data set, to determine (i)-(iii),
optionally wherein the one or more other similarly sourced plants include plants within a same zone as the plant.

3. The method of claim 1, wherein outputting, by the processor, the indication that the
plant has the latent infection and the one or more determined operations comprises:
generating an alert message that, when processed by a user device, causes the user device to output an alert notifying a user of the user device to perform one or more of the determined operations; and
transmitting the generated alert message to the user device.

4. The method of claim 1, wherein determining, by the processor, the one or more
operations to mitigate the latent infection in the plant comprises:
determining, based on the generated output, that an anti-microbial treatment should be prescribed for one or more other similarly sourced plants pre-harvest.

5. The method of claim 4, wherein determining, by the processor, the one or more
operations to mitigate the latent infection in the plant comprises:
generating instructions that, when processed by an irrigation controller, cause the irrigation controller to automatically disperse a liquid including the anti-microbial treatment; and
transmitting the instructions to the irrigation controller.

6. The method of claim 1, wherein determining, by the processor, the one or more
operations to mitigate the latent infection in the plant comprises:
(a) generating instructions that, when processed by a robotic device, cause the robotic device to (i) navigate to a location of with the plant and (ii) disperse an anti-microbial treatment onto the plant and one or more other similarly sourced plants; and
transmitting the instructions to the robotic device; or
(b) generating instructions that, when processed by a robotic device, cause the robotic device to (i) navigate to a location of the plant and (ii) harvest one or more plant products from the plant and one or more other similarly sourced plants before an expected harvesting timeframe; and
transmitting the instructions to the robotic device.

7. The method of claim 1, wherein the obtained data is generated by a nucleic acid sequencer based on sequencing plant products that are extracted from the plant.

8. The method of claim 7, wherein the plant products are nondestructively sampled from the plant.

9. The method of claim 7, wherein the plant products include volatile components off-gassed by the plant.

10. The method of claim 1, wherein the one or more machine learning models include at least one of a binary logistic regression model, logistic model tree, random forest classifier, L2 regularization, partial least squares, and convolutional neural networks, CNNs.

11. The method of claim 1, further comprising:
encoding, by the processor, the obtained data into a data structure for input to the one or more machine learning models; and
providing, by the processor, the encoded data structure as input to the one or more machine learning models.

12. The method of claim 1, further comprising performing, by the processor, one or more of the determined operations to mitigate the latent infection in the plant.

13. The method of claim 1, wherein selecting, by the processor, one or more machine learning models is further based on one or more prediction features identified from the obtained data, the one or more prediction features including at least one of a growing region of the plant, environmental conditions, plant type, stage of growth of the plant, non-invasive measurements of the plant, invasive measurements of the plant, dry matter content, gene expression, outgassed volatile components of the plant, a growing zone, and read sequence data of the plant.

14. The method of claim 1, wherein the known plant information includes at least one of historic growing information about the one or more other plants, a growing season length, soil conditions, levels of precipitation, amounts of sunlight, environmental temperatures, a growing region, and a plant type.

15. A system for predicting a latent infection in a plant, the system comprising: one or more processors; and
one or more computer-readable storage devices having stored thereon instructions that, when executed by the one or more processors, cause the one or more processors to perform operations according to the method of any one of claims 1 to 14.

## Patentansprüche

1. Verfahren zum Identifizieren einer vor der Ernte vorliegenden latenten Infektion in einer Pflanze, wobei das Verfahren Folgendes umfasst:
Erlangen von Daten durch einen Prozessor, die ein Expressionsniveau eines oder mehrerer in der Pflanze vorhandener Infektionsbiomarker beschreiben, wobei die Daten eine Liste einer oder mehrerer Varianten einschließen, wobei die Infektionsbiomarker eine Wahrscheinlichkeit einer Infektion in der Pflanze angeben, wobei die eine oder die mehreren Varianten Unterschiede zwischen einer Lesesequenz der Pflanze und einem Referenzgenom einer gesunden Pflanze eines gleichen Typs wie die Pflanze beschreiben, wobei die Lesesequenz der Pflanze eine Reihenfolge von Nucleotiden in einer Nucleinsäure der Pflanze darstellt;
Auswählen eines oder mehrerer Modelle maschinellen Lernens durch den Prozessor basierend auf den erlangten Daten, wobei das eine oder die mehreren Modelle maschinellen Lernens zuvor unter Verwendung von Daten trainiert wurden, die andere Daten und einen bestimmten oder mehrere bestimmte Infektionsbiomarker einer oder mehrerer anderer Pflanzen korrelieren, um eine Ausgabe zu generieren, die eine Wahrscheinlichkeit angibt, dass die Pflanze vor der Ernte eine latente Infektion entwickelt, wobei das eine oder die mehreren Modelle maschinellen Lernens unter Verwendung eines Vorgangs trainiert wurden, der Folgendes umfasst:
Eingeben, aus einem Trainingsdatensatz und in das eine oder die mehreren Modelle maschinellen Lernens, von (i) nicht invasiven Messungen der einen oder der mehreren anderen Pflanzen, (ii) invasiven Messungen der einen oder der mehreren anderen Pflanzen, (iii) bekannten Pflanzeninformationen für die eine oder die mehreren anderen Pflanzen und (iv) bekräftigenden Infektionsidentifizierungen für die eine oder die mehreren anderen Pflanzen und
Bestimmen vorhergesagter Wahrscheinlichkeiten, dass die eine oder die mehreren anderen Pflanzen vor der Ernte die latente Infektion entwickeln, basierend auf dem Eingeben von (i)-(iv) in das eine oder die mehreren Modelle maschinellen Lernens und
Ausgeben des einen oder der mehreren Modelle maschinellen Lernens zur Laufzeitverwendung;
Generieren einer Ausgabe durch den Prozessor, die die Wahrscheinlichkeit angibt, dass die Pflanze vor der Ernte die latente Infektion entwickelt, basierend auf dem Anwenden des einen oder der mehreren Modelle maschinellen Lernens auf die Daten;
Bestimmen durch den Prozessor, dass die Pflanze eine latente Infektion aufweist, basierend darauf, dass die Ausgabe einen vorbestimmten Schwellenbereich überschreitet;
Bestimmen einer oder mehrerer Operationen durch den Prozessor, um die latente Infektion in der Pflanze einzudämmen; und
Ausgeben einer Angabe durch den Prozessor, dass die Pflanze die latente Infektion und die eine oder die mehreren bestimmten Operationen aufweist.

2. Verfahren nach Anspruch 1, wobei das Bestimmen der einen oder der mehreren Operationen durch den Prozessor, um die latente Infektion in der Pflanze einzudämmen, Bestimmen mindestens eines der Folgenden für die Pflanze und eine oder mehrere andere ähnlich bezogene Pflanzen umfasst: (i) eines modifizierten Erntezeitplans, der früher in einer Anbausaison liegt, (ii) Anweisungen zum Anwenden einer vorbestimmten Menge an Pestizid vor der Ernte und (iii) wann sich die Pflanze und die eine oder die mehreren anderen ähnlich bezogenen Pflanzen einem Ende einer jeweiligen gesunden Produktionslebensdauer nähern, wobei das eine oder die mehreren Modelle maschinellen Lernens zuvor unter Verwendung von Daten aus dem Trainingsdatensatz trainiert wurden, um (i)-(iii) zu bestimmen,
wobei optional die eine oder die mehreren anderen ähnlich bezogenen Pflanzen Pflanzen innerhalb einer gleichen Zone wie die Pflanze einschließen.

3. Verfahren nach Anspruch 1, wobei das Ausgeben der Angabe durch den Prozessor, dass die Pflanze die latente Infektion und die eine oder die mehreren bestimmten Operationen aufweist, Folgendes umfasst:
Generieren einer Alarmmeldung, die, wenn sie durch eine Benutzervorrichtung verarbeitet wird, die Benutzervorrichtung dazu veranlasst, einen Alarm auszugeben, der einen Benutzer der Benutzervorrichtung darüber benachrichtigt, eine oder mehrere der bestimmten Operationen durchzuführen; und
Übertragen der generierten Alarmmeldung an die Benutzervorrichtung.

4. Verfahren nach Anspruch 1, wobei das Bestimmen der einen oder der mehreren Operationen durch den Prozessor, um die latente Infektion in der Pflanze einzudämmen, Folgendes umfasst:
Bestimmen basierend auf der generierten Ausgabe, dass eine antimikrobielle Behandlung für eine oder mehrere andere ähnlich bezogene Pflanzen vor der Ernte verordnet werden sollte.

5. Verfahren nach Anspruch 4, wobei das Bestimmen der einen oder der mehreren Operationen durch den Prozessor, um die latente Infektion in der Pflanze einzudämmen, Folgendes umfasst:
Generieren von Anweisungen, die, wenn sie durch ein Bewässerungssteuergerät verarbeitet werden, das Bewässerungssteuergerät dazu veranlassen, automatisch eine Flüssigkeit zu verteilen, die die antimikrobielle Behandlung einschließt; und
Übertragen der Anweisungen an das Bewässerungssteuergerät.

6. Verfahren nach Anspruch 1, wobei das Bestimmen der einen oder der mehreren Operationen durch den Prozessor, um die latente Infektion in der Pflanze einzudämmen, Folgendes umfasst:
(a) Generieren von Anweisungen, die, wenn sie durch eine robotische Vorrichtung verarbeitet werden, die robotische Vorrichtung dazu veranlassen, (i) zu einem Standort von mit der Pflanze zu navigieren und (ii) eine antimikrobielle Behandlung auf der Pflanze und einer oder mehreren anderen ähnlich bezogenen Pflanzen zu verteilen; und
Übertragen der Anweisungen an die robotische Vorrichtung; oder
(b) Generieren von Anweisungen, die, wenn sie durch eine robotische Vorrichtung verarbeitet werden, die robotische Vorrichtung dazu veranlassen, (i) zu einem Standort der Pflanze zu navigieren und (ii) ein oder mehrere Pflanzenprodukte von der Pflanze und einer oder mehreren anderen ähnlich bezogenen Pflanzen vor einem erwarteten Erntezeitrahmen zu ernten; und
Übertragen der Anweisungen an die robotische Vorrichtung.

7. Verfahren nach Anspruch 1, wobei die erlangten Daten durch einen Nucleinsäuresequenzer basierend auf dem Sequenzieren von Pflanzenprodukten generiert werden, die aus der Pflanze extrahiert werden.

8. Verfahren nach Anspruch 7, wobei die Pflanzenprodukte zerstörungsfrei von der Pflanze beprobt werden.

9. Verfahren nach Anspruch 7, wobei die Pflanzenprodukte durch die Pflanze ausgegaste flüchtige Bestandteile einschließen.

10. Verfahren nach Anspruch 1, wobei das eine oder die mehreren Modelle maschinellen Lernens mindestens eines von einem binären logistischen Regressionsmodell, logistischen Modellbaum, Random-Forest-Klassifikator, L2-Regularisierung, partiellen kleinsten Quadraten und faltenden neuronalen Netzen, CNNs, einschließen.

11. Verfahren nach Anspruch 1, ferner umfassend:
Codieren der erlangten Daten durch den Prozessor in eine Datenstruktur zur Eingabe in das eine oder die mehreren Modelle maschinellen Lernens; und
Bereitstellen der codierten Datenstruktur durch den Prozessor als Eingabe in das eine oder die mehreren Modelle maschinellen Lernens.

12. Verfahren nach Anspruch 1, ferner umfassend Durchführen einer oder mehrerer der bestimmten Operationen durch den Prozessor, um die latente Infektion in der Pflanze einzudämmen.

13. Verfahren nach Anspruch 1, wobei das Auswählen eines oder mehrerer Modelle maschinellen Lernens durch den Prozessor ferner auf einem oder mehreren aus den erlangten Daten identifizierten Vorhersagemerkmalen basiert, wobei das eine oder die mehreren Vorhersagemerkmale mindestens eines von einer Anbauregion der Pflanze, Umgebungsbedingungen, Pflanzentyp, Wachstumsstadium der Pflanze, nichtinvasiven Messungen der Pflanze, invasiven Messungen der Pflanze, Trockenmassegehalt, Genexpression, ausgegasten flüchtigen Bestandteilen der Pflanze, einer Anbauzone und Lesesequenzdaten der Pflanze einschließen.

14. Verfahren nach Anspruch 1, wobei die bekannten Pflanzeninformationen mindestens eines von historischen Anbauinformationen über die eine oder die mehreren anderen Pflanzen, einer Länge der Anbausaison, Bodenbedingungen, Niederschlagsniveaus, Sonneneinstrahlungsmengen, Umgebungstemperaturen, einer Anbauregion und einem Pflanzentyp einschließen.

15. System zum Vorhersagen einer latenten Infektion in einer Pflanze, wobei das System Folgendes umfasst:
einen oder mehrere Prozessoren; und
eine oder mehrere computerlesbare Speichervorrichtungen, auf denen Anweisungen gespeichert sind, die, wenn sie durch den einen oder die mehreren Prozessoren ausgeführt werden, den einen oder die mehreren Prozessoren dazu veranlassen, Operationen gemäß dem Verfahren nach einem der Ansprüche 1 bis 14 durchzuführen.

## Revendications

1. Procédé permettant l'identification d'une infection latente avant récolte chez une plante, le procédé comprenant :
l'obtention, par un processeur, de données décrivant un niveau d'expression d'un ou plusieurs biomarqueurs d'infection présents chez la plante, les données comprenant une liste d'un ou plusieurs variants, les biomarqueurs d'infection indiquant une probabilité d'infection chez la plante, lesdits un ou plusieurs variants décrivant des différences entre une séquence de lecture de la plante et un génome de référence d'une plante saine du même type que la plante, ladite séquence de lecture de la plante représentant un ordre de nucléotides dans un acide nucléique de la plante ;
la sélection, par le processeur, d'un ou plusieurs modèles d'apprentissage automatique sur la base des données obtenues, lesdits un ou plusieurs modèles d'apprentissage automatique ayant été préalablement entraînés, à l'aide de données qui mettent en corrélation d'autres données et ont déterminé un ou plusieurs biomarqueurs d'infection d'une ou plusieurs autres plantes, pour générer une sortie indiquant une probabilité que la plante est en train de développer une infection latente avant récolte, lesdits un ou plusieurs modèles d'apprentissage automatique ayant été entraînés à l'aide d'un processus comprenant :
l'entrée, à partir d'un ensemble de données d'entraînement et dans lesdits un ou plusieurs modèles d'apprentissage automatique, (i) de mesures non invasives desdites une ou plusieurs autres plantes, (ii) de mesures invasives desdites une ou plusieurs autres plantes, (iii) d'informations connues sur les plantes pour lesdites une ou plusieurs autres plantes, et (iv) d'identifications d'infection affirmatives pour lesdites une ou plusieurs autres plantes, et
la détermination de probabilités prédites que lesdites une ou plusieurs autres plantes sont en train de développer l'infection latente avant récolte sur la base de la saisie (i) à (iv) dans lesdits un ou plusieurs modèles d'apprentissage automatique, et
la sortie de l'un ou plusieurs modèles d'apprentissage automatique pour une utilisation pendant l'exécution ;
la génération, par le processeur, d'une sortie indiquant la probabilité que la plante soit en train de développer l'infection latente avant récolte sur la base de l'application desdits un ou plusieurs modèles d'apprentissage automatique sur les données ;
la détermination, par le processeur, que la plante présente une infection latente sur la base de la sortie dépassant une plage de seuil prédéfinie ;
la détermination, par le processeur, d'une ou plusieurs opérations pour atténuer l'infection latente chez la plante ; et
la sortie, par le processeur, d'une indication que la plante présente l'infection latente et lesdites une ou plusieurs opérations déterminées.

2. Procédé selon la revendication 1, ladite détermination, par le processeur, desdites une ou plusieurs opérations destinées à atténuer l'infection latente chez la plante comprenant la détermination, pour la plante et une ou plusieurs autres plantes de source similaire, d'au moins un parmi (i) un calendrier de récolte modifié qui est plus tôt dans une saison de croissance, (ii) des instructions pour appliquer une quantité prédéfinie de pesticide avant récolte, et (iii) lorsque la plante et lesdites une ou plusieurs autres plantes de source similaire approchent une fin de durée de vie de production saine respective, lesdits un ou plusieurs modèles d'apprentissage automatique ayant été préalablement entraînés, à l'aide de données provenant de l'ensemble de données d'entraînement, pour déterminer (i) à (iii), éventuellement lesdites une ou plusieurs autres plantes de source similaire comprenant des plantes dans une même zone que la plante.

3. Procédé selon la revendication 1, ladite sortie, par le processeur, de l'indication selon laquelle la plante présente l'infection latente et lesdites une ou plusieurs opérations déterminées comprenant :
la génération d'un message d'alerte qui, lorsqu'il est traité par un dispositif utilisateur, amène le dispositif utilisateur à émettre une alerte notifiant à un utilisateur du dispositif utilisateur de réaliser une ou plusieurs des opérations déterminées ; et
la transmission du message d'alerte généré au dispositif utilisateur.

4. Procédé selon la revendication 1, ladite détermination, par le processeur, desdites une ou plusieurs opérations permettant d'atténuer l'infection latente chez la plante comprenant :
la détermination, en fonction des résultats générés, qu'un traitement antimicrobien doit être prescrit pour une ou plusieurs autres plantes provenant de sources similaires avant récolte.

5. Procédé selon la revendication 4, ladite détermination, par le processeur, desdites une ou plusieurs opérations permettant d'atténuer l'infection latente chez la plante comprenant :
la génération d'instructions qui, lorsqu'elles sont traitées par un dispositif de commande d'irrigation, amènent le dispositif de commande d'irrigation à disperser automatiquement un liquide comprenant le traitement antimicrobien ; et
la transmission des instructions au dispositif de commande d'irrigation.

6. Procédé selon la revendication 1, ladite détermination, par le processeur, desdites une ou plusieurs opérations permettant d'atténuer l'infection latente chez la plante comprenant :
(a) la génération d'instructions qui, lorsqu'elles sont traitées par un dispositif robotisé, amènent le dispositif robotisé à (i) naviguer vers un emplacement avec la plante et (ii) disperser un traitement antimicrobien sur la plante et une ou plusieurs autres plantes de source similaire ; et
la transmission des instructions au dispositif robotisé ; ou
(b) la génération d'instructions qui, lorsqu'elles sont traitées par un dispositif robotisé, amènent le dispositif robotisé à (i) naviguer vers un emplacement de la plante et (ii) récolter un ou plusieurs produits végétaux de la plante et une ou plusieurs autres plantes de source similaire avant un délai de récolte prévu ; et
la transmission des instructions au dispositif robotisé.

7. Procédé selon la revendication 1, lesdites données obtenues étant générées par un séquenceur d'acide nucléique basé sur le séquençage de produits végétaux qui sont extraits de la plante.

8. Procédé selon la revendication 7, lesdits produits végétaux étant échantillonnés de manière non destructive à partir de la plante.

9. Procédé selon la revendication 7, lesdits produits végétaux comprenant des composants volatils dégagés par la plante.

10. Procédé selon la revendication 1, lesdits un ou plusieurs modèles d'apprentissage automatique comprenant au moins un parmi un modèle de régression logistique binaire, un arbre de modèle logistique, un classificateur de forêt aléatoire, une régularisation L2, les moindres carrés partiels et les réseaux neuronaux convolutifs, CNN.

11. Procédé selon la revendication 1, comprenant en outre :
le codage, par le processeur, des données obtenues dans une structure de données destinée à être entrée dans un ou plusieurs modèles d'apprentissage automatique ; et
la fourniture, par le processeur, de la structure de données codée en tant qu'entrée dans un ou plusieurs modèles d'apprentissage automatique.

12. Procédé selon la revendication 1, comprenant en outre la réalisation, par le processeur, d'une ou plusieurs des opérations déterminées pour atténuer l'infection latente chez la plante.

13. Procédé selon la revendication 1, ladite sélection, par le processeur, d'un ou plusieurs modèles d'apprentissage automatique étant en outre basée sur une ou plusieurs caractéristiques de prédiction identifiées à partir des données obtenues, lesdites une ou plusieurs caractéristiques de prédiction comprenant au moins un parmi une région de croissance de la plante, les conditions environnementales, le type de plante, le stade de croissance de la plante, les mesures non invasives de la plante, les mesures invasives de la plante, la teneur en matière sèche, l'expression génétique, les composants volatils dégagés de la plante, une zone de croissance et les données de séquence de lecture de la plante.

14. Procédé selon la revendication 1, lesdites informations connues sur les plantes comprenant au moins une parmi les informations de croissance historiques concernant une ou plusieurs autres plantes, une durée de saison de croissance, les conditions de sol, les niveaux de précipitations, les quantités de lumière solaire, des températures environnementales, une région de croissance et un type de plante.

15. Système permettant la prédiction d'une infection latente chez une plante, le système comprenant :
un ou plusieurs processeurs ; et
un ou plusieurs supports lisibles par ordinateur stockant des instructions qui, lorsqu'elles sont exécutées par lesdits un ou plusieurs processeurs, amènent lesdits un ou plusieurs processeurs à réaliser des opérations selon le procédé selon l'une quelconque des revendications 1 à 14.
